# EUROPEAN PATENT APPLICATION

(11) **EP 1 514 870 A1**
(43) Date of publication of application: **16.03.2005**
(21) Application number: 03733425.7
(22) Date of filing: 13.06.2003
(51) Int. Cl.: C07D 233/54, C07D 401/12, C07D 405/04, C07D 405/14, C07D 471/04, A61K 31/4439, A61K 31/506, A61K 31/513

(54) **PRODRUG AND PROCESS FOR PRODUCING THE SAME**

(30) Priority: 14.06.2002 JP 2002175086; 19.02.2003 JP 2003041085
(71) Applicant: Takeda Pharmaceutical Company Limited, Osaka 541-0045 (JP)
(72) Inventor: KAMIYAMA, Keiji, Ibaraki-shi, Osaka 567-0037 (JP); BANNO, Hiroshi, Kawanishi-shi, Hyogo 666-0004 (JP); SATO, Fumihiko, Suita-shi, Osaka 565-0872 (JP); HASUOKA, Atsushi, Takatsuki-shi, Osaka 569-0043 (JP)
(74) Representative: Lewin, John Harvey
(86) International application number: PCT/JP2003/007545
(87) International publication number: WO 2003/106429

(57) **Abstract**

The present invention provides a compound having, as a modification group to be eliminated from a prodrug, a group represented by the formula: 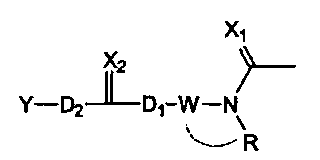 wherein each symbol is as defined in the specification. According to the present invention, the development of a prodrug based on the modification of a nitrogen-containing heterocycle and the like has become possible.

## Description

### Technical Field to Which the Invention Pertains

The present invention relates to a novel prodrug and a production method thereof.

### Background Art

Inasmuch as the absorbability of an efficacious component of a pharmaceutical agent in the living body is an important factor that influences expression, intensity and duration of pharmacological action, any pharmaceutical agent is required to show stable and good absorbability.

Conventionally, designs for producing preparations such as processing into a fine powder or an amorphous form, use of an emulsifier and the like for the purpose of improving absorbability of a pharmaceutically active ingredient (hereinafter sometimes to be referred to as a pharmaceutical compound or a therapeutic drug) have been examined. However, processing into a fine powder or an amorphous form is sometimes restricted in application, depending on the characteristics (low melting point, hygroscopicity, low stability and the like) of a pharmaceutical compound, and the use of an emulsifier represented by castor oil and the like may cause hypotension, dyspnea or hyperreaction such as urticaria and the like.

Besides the above-mentioned designs for producing a preparation, as an attempt to enhance the solubility and improve the absorbability of a pharmaceutical compound, processing a pharmaceutical compound into a prodrug has been considered. A prodrug is defined to be a compound pharmacologically inactive but converted to a pharmaceutical compound that exhibits a pharmacological activity by the action of enzyme and the like in the living body or during the absorption process in the living body.

By the adoption of the method of forming a prodrug, not only the absorbability of a pharmaceutical compound can be improved but also effects such as improvement of chemical stability, regulation of the level of pharmacological action, prolongation of pharmacological action, reduction of side effects and the like can be expected. Therefore, forming a prodrug has become an important method for the development of pharmaceutical products.

For forming a prodrug, esterification of a carboxyl group, acylation, carbonic acid esterification or carbamoylation of a hydroxyl group, and the like of a pharmaceutical compound have been widely used. However, pharmaceutical compounds to be formed into prodrugs do not necessarily have a functional group such as a carboxyl group, a hydroxyl group and the like.

A nitrogen-containing heterocycle has been often recognized as a partial structure of a compound having a pharmacological action, and moreover, a nitrogen-containing heterocycle sometimes forms a structure of a fused ring. In view of the fact that many useful therapeutic drugs having a nitrogen-containing heterocycle as a partial structure are known, a pharmacological importance of this partial structure can be appreciated. On the other hand, many of the compounds having a nitrogen-containing heterocycle show poor absorbability because of poor solubility and the like. Consequently, many such compounds could not be put to practice because of absorbability, even if usefulness as pharmaceutical compounds can be expected from their pharmacological actions.

While examples of formation of a prodrug based on the modification of a nitrogen-containing heterocycle are very small in number as compared to the above-mentioned formation of a prodrug by modification of carboxyl group or hydroxyl group, US Patent Nos. 5,021,433; 4,873,337; 6,093,734; 4,045,563; 4,686,230; 4,873,337; 4,965,269; 5,021,433; 5,039,806 and the like disclose examples of such prodrug.

However, known prodrugs include many problematic prodrugs for general application as prodrugs of a wide range of pharmaceutical compounds, because they are associated with problems in terms of safety such as generation of formaldehyde, acetaldehyde and the like during the reproductive process of the original pharmaceutical compound (i.e., parent compound) in the living body, insufficient absorbability, incomplete reproduction of parent pharmaceutical compound and the like.

While T.W. Greene, P.G.M. Wuts, Protective Groups In Organic Synthesis, second edition, John Wiley & Sons, pp. 385-397 describes N-sulfonylation, carbamation, N-alkylation and the like as a method for introducing a protecting group into azoles represented by imidazole, application of any of these to a prodrug is difficult in consideration of the chemical stability of a derivative. In addition, as a requirement a prodrug should fulfill, reproduction of a pharmaceutical compound in the living body or during the absorbing process in the living body can be mentioned. However, it is considered to be difficult to exert sufficient action of enzyme on a derivative, wherein a protecting group has been introduced into azoles, and rapidly achieve reproduction of a pharmaceutical compound (parent compound).

As mentioned above, the technique for forming a prodrug based on the modification of a nitrogen-containing heterocycle at the present stage is not entirely sufficient. Once such a technique for forming a prodrug is developed, a compound having a useful pharmacological action, which could not be put to practical use due to low absorbability and the like, may be developed as a pharmaceutical product.

The present invention has been made in such situation, and provides development of a novel prodrug and a means therefor.

### Disclosure of the Invention

The present inventors have conducted intensive studies in an attempt to develop a prodrug based on the modification of a nitrogen-containing heterocycle and to find a means therefor, and as a result, found usefulness of not only a pharmaceutical compound useful as a prophylactic or therapeutic drug, which has a nitrogen-containing heterocycle as a partial structure (hereinafter sometimes to be simply referred to as a therapeutic drug), but also a compound represented by the following formula (I) as a prodrug of a therapeutic drug having other eliminatable proton as a partial structure thereof, which resulted in the completion of the present invention.

Accordingly, the present invention provides the following.
1) A prodrug compound having, as a modification group to be eliminated from the prodrug, a group represented by the formula: 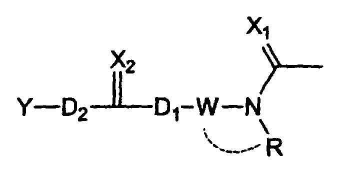 wherein
   - X₁ and X₂: are each an oxygen atom or a sulfur atom,
   - W: is a chain divalent hydrocarbon group optionally having substituent(s) or a divalent group represented by the formula:

   -W₁-Z-W₂-

   wherein W₁ and W₂ are each a chain divalent hydrocarbon group or a bond, Z is a divalent hydrocarbon ring group optionally having substituent(s), a divalent heterocyclic group optionally having substituent(s), an oxygen atom, SOₙ wherein n is 0, 1 or 2, or >N-E wherein E is a hydrogen atom, a hydrocarbon group optionally having substituent(s), a heterocyclic group optionally having substituent(s), a lower alkanoyl group, a lower alkoxycarbonyl group, an aralkyloxycarbonyl group, a thiocarbamoyl group, a lower alkylsulfinyl group, a lower alkylsulfonyl group, a sulfamoyl group, a mono-lower alkylsulfamoyl group, a di-lower alkylsulfamoyl group, an arylsulfamoyl group, an arylsulfinyl group, an arylsulfonyl group, an arylcarbonyl group or a carbamoyl group optionally having substituent(s), and when Z is an oxygen atom, SOₙ or >N-E, W₁ and W₂ are each a chain divalent hydrocarbon group,
   - R: is a hydrogen atom, a hydrocarbon group optionally having substituent(s) or a heterocyclic group optionally having substituent(s), and
   - R and W: may be bonded to each other when R is not a hydrogen atom,
   - D₁ and D₂: are each a bond, an oxygen atom, a sulfur atom or >NR₁ wherein R₁ is a hydrogen atom or a hydrocarbon group optionally having substituent(s), except for when both D₁ and D₂ are bonds, and
   - Y: is a hydrocarbon group optionally having substituent(s) or a heterocyclic group optionally having substituent(s).
2) The compound of the above-mentioned 1), which is a compound represented by the formula (I): 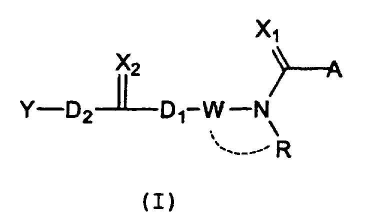 wherein A is a group remaining from elimination of hydrogen from a parent compound H-A of a prodrug having a group capable of bonding to a carbon atom of a modification group (hereinafter sometimes to be simply referred to as a side chain) eliminatable from a prodrug, via a carbon-oxygen bond, a carbon-sulfur bond or a carbon-nitrogen bond, and other symbols are as defined in the above-mentioned 1), or a salt thereof.
3) The compound of the above-mentioned 1), wherein R is a hydrocarbon group optionally having substituent(s) or a heterocyclic group optionally having substituent(s).
4) The compound of the above-mentioned 1), wherein Z is a divalent hydrocarbon ring group optionally having substituent(s) or a divalent heterocyclic group optionally having substituent(s).
5) The compound of the above-mentioned 1), wherein X₁ and X₂ are each an oxygen atom.
6) The compound of the above-mentioned 1), wherein D₁ and D₂ are each a bond or an oxygen atom, except for when both D₁ and D₂ are bonds.
7) The compound of the above-mentioned 1), wherein W is a chain divalent hydrocarbon group optionally having substituent(s).
8) The compound of the above-mentioned 1), wherein W is an ethylene group.
9) The compound of the above-mentioned 1), wherein R is a C₁₋₆ hydrocarbon group optionally having substituent(s).
10) The compound of the above-mentioned 1), wherein Y is a C₁₋₆ hydrocarbon group optionally having substituent(s) or a saturated heterocyclic group optionally having substituent(s), which contains, as ring-constituting atom, 1 to 4 heteroatom(s) selected from oxygen atom, nitrogen atom and sulfur atom.
11) The compound of the above-mentioned 1), wherein X₁ and X₂ are each an oxygen atom, D₁ and D₂ are each a bond or an oxygen atom except for when both D₁ and D₂ are bonds, W is an ethylene group, R is a C₁₋₆ alkyl group, and Y is a C₁₋₆ hydrocarbon group optionally having substituent(s) or a saturated oxygen-containing heterocyclic group optionally having substituent(s), which may further contain, as ring-constituting atom, 1 to 3 heteroatom(s) selected from oxygen atom, nitrogen atom and sulfur atom.
12) The compound of the above-mentioned 1), which is a compound represented by the formula (II): 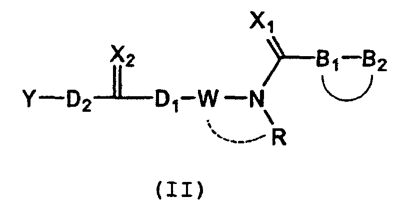 wherein -B₁-B₂ is a group remaining from elimination of hydrogen from a pharmaceutical compound H-B₁-B₂ wherein H-B₁- is a hydroxyl group, a thiol group, an amide group or an optionally fused, nitrogen-containing heterocycle optionally having substituent(s), which is capable of bonding to a carbon atom of a modification group (side chain) eliminatable from a prodrug, via a carbon-oxygen bond, a carbon-sulfur bond or a carbon-nitrogen bond, and other symbols are as defined in the above-mentioned 1),
   or a salt thereof.
13) The compound of the above-mentioned 12), wherein B₁ is an optionally fused, nitrogen-containing heterocyclic group optionally having substituent(s), which is capable of bonding to a carbon atom of a modification group (side chain) eliminatable from a prodrug, via a carbon-nitrogen bond.
14) The compound of the above-mentioned 13), wherein the nitrogen-containing heterocyclic group represented by B₁ is a 5 or 6-membered aromatic heterocyclic group containing 1 to 4 nitrogens.
15) The compound of the above-mentioned 14), wherein the aromatic heterocycle in the 5 or 6-membered aromatic heterocyclic group containing 1 to 4 nitrogens, which is represented by B₁, is imidazole, pyrrole, pyrazole, isoxazole, oxazole, thiazole or triazole.
16) (1) A production method of the compound of the above-mentioned 2), which comprises reacting a pharmaceutical compound having an eliminatable proton (H) represented by the formula (III):

   H-A (III)

   or a salt thereof with a compound represented by the formula (IV) : 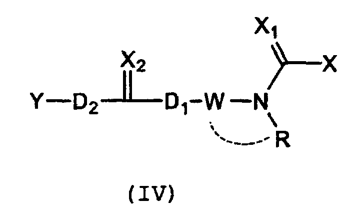 wherein X is a leaving group, and other symbols are as defined in the above-mentioned 1), or a salt thereof, or a compound of the formula (V): 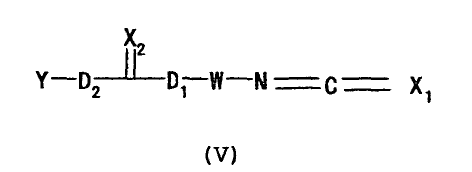 wherein each symbol is as defined in the above-mentioned 1),
   or a salt thereof.
17) A compound represented by the formula (VI): 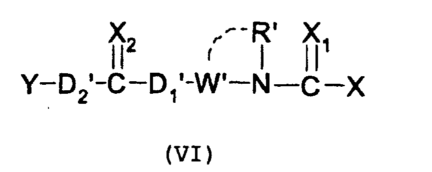 wherein X₁ and X₂ are each an oxygen atom or a sulfur atom, W' is a chain divalent hydrocarbon group having 2 or more carbon atoms and optionally having substituent(s), or a divalent group represented by the formula:

   -W₁'-Z'-W₂'-

   wherein W₁' and W₂' are each a chain divalent hydrocarbon group or bond, Z' is a divalent hydrocarbon ring group optionally having substituent(s) or a divalent heterocyclic group optionally having substituent(s), R' is a hydrocarbon group optionally having substituent(s) or a heterocyclic group optionally having substituent(s), R' is optionally bonded to W', D₁' is an oxygen atom or a sulfur atom and D₂' is an oxygen atom, or D₁' is a sulfur atom and D₂' is a bond, Y is a hydrocarbon group optionally having substituent(s) or a heterocyclic group optionally having substituent(s), and X is a leaving group, or a salt thereof.
18) Use of a compound represented by the formula (IV): 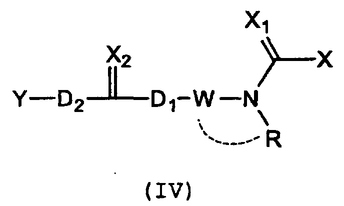 wherein X is a leaving group, and other symbols are as defined in the above-mentioned 1), for the production of a prodrug compound or a salt thereof.
19) Use of a compound of the formula (V): 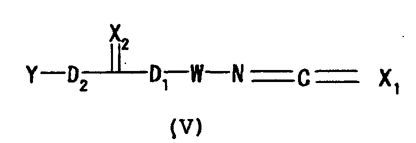
wherein each symbol is as defined in the above-mentioned 1), for the production of a prodrug compound or a salt thereof.

### Embodiment of the Invention

In the present invention, X₁ and X₂ represent an oxygen atom and a sulfur atom, respectively. Both X₁ and X₂ preferably represent an oxygen atom.

In the present invention, W represents a "chain divalent hydrocarbon group optionally having substituent(s)", or the formula:

-W₁-Z-W₂-

wherein W₁ and W₂ are each a "chain divalent hydrocarbon group" or a bond, and Z is a divalent group such as a "divalent hydrocarbon ring group optionally having substituent(s)", a "divalent heterocyclic group optionally having substituent(s)", an oxygen atom, SOₙ wherein n is 0, 1 or 2 or >N-E wherein E is a hydrogen atom, a hydrocarbon group optionally having substituent(s), a heterocyclic group optionally having substituent(s), a lower alkanoyl group, a lower alkoxycarbonyl group, an aralkyloxycarbonyl group, a thiocarbamoyl group, a lower alkylsulfinyl group, a lower alkylsulfonyl group, a sulfamoyl group, a mono-lower alkylsulfamoyl group, a di-lower alkylsulfamoyl group, an arylsulfamoyl group, an arylsulfinyl group, an arylsulfonyl group, an arylcarbonyl group, or a carbamoyl group optionally having substituent(s), when Z is an oxygen atom, SOₙ or >N-E, W₁ and W₂ are each a "chain divalent hydrocarbon group". Particularly, W is preferably a "chain divalent hydrocarbon group optionally having substituent(s)".

In the present invention, W' is a "chain divalent hydrocarbon group optionally having substituent(s)", or divalent group represented by the formula:

-W₁'-Z'-W₂'-

wherein W₁' and W₂' are each a "chain divalent hydrocarbon group" or a bond, and Z' is a "divalent hydrocarbon ring group optionally having substituent(s)" or a "divalent heterocyclic group optionally having substituent(s)". Of these, W' is preferably a "chain divalent hydrocarbon group optionally having substituent(s)".

As the "chain divalent hydrocarbon group" of the "chain divalent hydrocarbon group optionally having substituent(s)" represented by W, W' and "chain divalent hydrocarbon group" represented by W₁, W₁', W₂ and W₂', for example, a C₁₋₆ alkylene group (e.g., methylene, ethylene, trimethylene etc.), a C₂₋₆ alkenylene group (e.g., ethenylene etc.), a C₂₋₆ alkynylene group (e.g., ethynylene etc.) and the like can be mentioned. The chain divalent hydrocarbon group for W and W' may have 1 to 6 substituents similar to those for the "benzene ring optionally having substituent(s)" represented by ring B to be mentioned below at substitutable positions thereof.

As the "chain divalent hydrocarbon group" of the "chain divalent hydrocarbon group optionally having substituent(s)" represented by W and W' and "chain divalent hydrocarbon group" represented by W₁, W₁', W₂ and W₂', a methylene group and an ethylene group are preferable. As W and W', an ethylene group is particularly preferable. When Z is an oxygen atom, SOₙ or >N-E (n and E are as defined above), the "chain divalent hydrocarbon group" represented by W₁ is preferably a hydrocarbon group having 2 or more carbon atoms.

As the "hydrocarbon ring" of the "divalent hydrocarbon ring group optionally having substituent(s)" represented by Z and Z', for example, an alicyclic hydrocarbon ring, an aromatic hydrocarbon ring and the like can be mentioned, with preference given to one having 3 to 16 carbon atoms, which may have 1 to 4 substituents similar to those for the "benzene ring optionally having substituent(s)" represented by ring B at substitutable positions thereof. As the hydrocarbon ring, for example, cycloalkane, cycloalkene, arene and the like are used.

As a cycloalkane in the "divalent hydrocarbon ring group optionally having substituent(s)" represented by Z and Z', for example, a lower cycloalkane and the like are preferable, and, for example, C₃₋₁₀ cycloalkane such as cyclopropane, cyclobutane, cyclopentane, cyclohexane, cycloheptane, cyclooctane, bicyclo[2.2.1]heptane, adamantane etc., and the like are generally used.

As a cycloalkene in the "divalent hydrocarbon ring group optionally having substituent(s)" represented by Z and Z', for example, a lower cycloalkene is preferable, and, for example, C₄₋₉ cycloalkene such as cyclopropene, cyclobutene, cyclopentene, cyclohexene, cycloheptene, cyclooctene etc., and the like are generally used.

As an arene in the "divalent hydrocarbon ring group optionally having substituent(s)" represented by Z and Z', for example, a C₆₋₁₄ arene such as benzene, naphthalene, phenanthrene etc., and the like are preferable, and, for example, phenylene and the like are generally used.

As a heterocycle in the "divalent heterocyclic group optionally having substituent(s)" represented by Z and Z', a 5- to 12-membered "aromatic heterocycle" or "saturated or unsaturated non-aromatic heterocycle" containing, as ring-constituting atom (ring atom), 1 to 3 (preferably 1 or 2) kinds of at least 1 (preferably 1 to 4, more preferably 1 or 2) hetero atoms selected from oxygen atom, sulfur atom and nitrogen atom etc., and the like can be mentioned, which may have 1 to 4 substituents similar to those for the "benzene ring optionally having substituent(s)" represented by ring B to be mentioned below at substitutable positions thereof.

As an aromatic heterocycle in the "divalent heterocyclic group optionally having substituent(s)" represented by Z and Z', an aromatic monocyclic heterocycle, a fused aromatic heterocycle and the like can be mentioned.

As the "aromatic monocyclic heterocycle", for example, a 5- or 6-membered aromatic monocyclic heterocycle such as furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazan, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine etc., and the like can be mentioned.

As the "fused aromatic heterocycle", for example, a 8- to 12-membered fused aromatic heterocycle such as benzofuran, isobenzofuran, benzothiophene, isobenzothiophene, indole, isoindole, 1H-indazole, benzimidazole, benzoxazole, 1,2-benzisoxazole, benzothiazole, 1,2-benzisothiazole, 1H-benzotriazole, quinoline, isoquinoline, cinnoline, quinazoline, quinoxaline, phthalazine, naphthyridine, purine, pteridine, carbazole, carboline, acridine, phenoxazine, phenothiazine, phenazine, phenoxathiin, thianthrene, phenanthridine, phenanthroline, indolizine, pyrrolo[1,2-b]pyridazine, pyrazolo[1,5-a]pyridine, imidazo[1,2-a]pyridine, imidazo[1,5-a]pyridine, imidazo[1,2-b]pyridazine, imidazo[1,2-a]pyrimidine, 1,2,4-triazolo[4,3-a]pyridine, 1,2,4-triazolo[4,3-b]pyridazine etc., and the like can be mentioned.

As a saturated or unsaturated non-aromatic heterocycle in the "divalent heterocyclic group optionally having substituent(s)" represented by Z and Z', for example, a 3- to 8-membered (preferably 5- or 6-membered) saturated or unsaturated (preferably saturated) non-aromatic heterocycle (aliphatic heterocycle) such as oxirane, azetidine, oxetane, thietane, pyrrolidine, tetrahydrofuran, tetrahydrothiophene, piperidine, tetrahydropyran, tetrahydrothiopyran, morpholine, thiomorpholine, piperazine, azepane, oxepane, thiene, oxazepane, thiazepane, azocane, oxocane, thiocane, oxazocane, thiazocane etc., and the like can be mentioned. These may be oxo-substituted and may be, for example, 2-oxoazetidine, 2-oxopyrrolidine, 2-oxopiperidine, 2-oxoazepane, 2-oxoazocane, 2-oxotetrahydrofuran, 2-oxotetrahydropyran, 2-oxotetrahydrothiophene, 2-oxothiane, 2-oxopiperazine, 2-oxooxepane, 2-oxooxazepane, 2-oxothiepane, 2-oxothiazepane, 2-oxooxocane, 2-oxothiocane, 2-oxooxazocane, 2-oxothiazocane and the like.

The two bonds from the "hydrocarbon ring group" of the "divalent hydrocarbon ring group optionally having substituent(s)" or the "heterocyclic group" of the "divalent heterocyclic group optionally having substituent(s)" represented by Z and Z' may be present at any bondable position.

The "hydrocarbon group optionally having substituent(s)" and "heterocyclic group optionally having substituent(s)" represented by E is as defined in the following.

As the "lower alkanoyl group" represented by E, for example, formyl, a C₁₋₆ alkyl-carbonyl group such as acetyl, propionyl, butyryl, isobutyryl etc., and the like can be used.

As the "lower alkoxycarbonyl group" represented by E, for example, a C₁₋₆ alkoxy-carbonyl group such as methoxycarbonyl, ethoxycarbonyl, propoxycarbonyl, butoxycarbonyl etc., and the like are used.

As the "aralkyloxycarbonyl" represented by E, for example, a C₇₋₁₁ aralkyloxy-carbonyl group such as benzyloxycarbonyl etc., and the like are used.

As the "lower alkylsulfinyl group" represented by E, for example, a C₁₋₆ alkylsulfinyl group such as methylsulfinyl, ethylsulfinyl etc., and the like are used.

As the "lower alkylsulfonyl group" represented by E, for example, a C₁₋₆ alkylsulfonyl group such as methylsulfonyl, ethylsulfonyl etc., and the like are used.

As the "mono-lower alkylsulfamoyl group" represented by E, for example, a mono-C₁₋₆ alkylsulfamoyl group such as methylsulfamoyl, ethylsulfamoyl etc., and the like are used.

As the "di-lower alkylsulfamoyl group" represented by E, for example, a di-C₁₋₆ alkylsulfamoyl group such as dimethylsulfamoyl, diethylsulfamoyl etc., and the like are used.

As the "arylsulfamoyl group" represented by E, for example, a C₆₋₁₀ arylsulfamoyl group such as phenylsulfamoyl, naphthylsulfamoyl etc., and the like are used.

As the "arylsulfinyl group" represented by E, for example, a C₆₋₁₀ arylsulfinyl group such as phenylsulfinyl, naphthylsulfinyl etc., and the like are used.

As the "arylsulfonyl group" represented by E, for example, a C₆₋₁₀ arylsulfonyl group such as phenylsulfonyl, naphthylsulfonyl etc., and the like are used.

As the "arylcarbonyl group" represented by E, for example, C₆₋₁₀ aryl-carbonyl group such as benzoyl, naphthoyl etc., and the like are used.

The "carbamoyl group optionally having substituent(s)" represented by E is, for example, a group of the formula -CONR₂R₃ wherein R₂ and R₃ are each a hydrogen atom, a hydrocarbon group optionally having substituent(s) or a heterocyclic group optionally having substituent(s), and in the formula -CONR₂R₃, R₂ and R₃ may form a ring together with the adjacent nitrogen atom, and the like.

In the present invention, R is a hydrogen atom, a "hydrocarbon group optionally having substituent(s)" or a "heterocyclic group optionally having substituent(s)", R' is "a hydrocarbon group optionally having substituent(s)" or a "heterocyclic group optionally having substituent(s)". R can be bonded to W and R' can be bonded to W'. Of these, a C₁₋₆ hydrocarbon group optionally having substituent(s) is preferable and a lower (C₁₋₆) alkyl group is particularly preferable. The "hydrocarbon group optionally having substituent(s)" and "heterocyclic group optionally having substituent(s)" represented by R and R' are as defined in the following. A detailed explanation of the case where R is bonded to W and where R' is bonded to W' is given in the following.

In the present invention, D₁ and D₂ are each a bond, an oxygen atom, a sulfur atom or >NR₁, and in the formula, R₁ is a hydrogen atom or a hydrocarbon group optionally having substituent(s). However, the present invention excludes a case where D₁ and D₂ are both respectively a bond. Among others, each of D₁ and D₂ is preferably a bond or an oxygen atom, and particularly preferably, D₁ is an oxygen atom and D₂ is an oxygen atom or a bond. The "hydrocarbon group optionally having substituent(s)" represented by R₁ is as defined in the following.

In the present invention, for D₁' and D₂', D₁' is an oxygen atom or a sulfur atom and D₂' is an oxygen atom, or D₁' is a sulfur atom and D₂' is a bond.

In the present invention, Y is a "hydrocarbon group optionally having substituent(s)" or a "heterocyclic group optionally having substituent(s)". Of these, a C₁₋₆ hydrocarbon group optionally having substituent(s) or a saturated heterocyclic group optionally having substituent(s), which contains, as ring-constituting atom, 1 to 4 hetero atoms selected from oxygen atom, sulfur atom and nitrogen atom is preferable. As Y, among others, a C₁₋₆ hydrocarbon group optionally having substituent(s) or a saturated oxygen-containing heterocyclic group optionally having substituent(s), which further contains, as ring-constituting atom, 1 to 3 hetero atoms selected from oxygen atom, sulfur atom and nitrogen atom is preferable. The "hydrocarbon group optionally having substituent(s)" and "heterocyclic group optionally having substituent(s)" represented by Y are as defined in the following.

As the "hydrocarbon group" of the "hydrocarbon group optionally having substituent(s)" represented by the above-mentioned E, R, R', R₁ and Y, for example, a saturated or unsaturated aliphatic hydrocarbon group, a saturated or unsaturated alicyclic hydrocarbon group, a saturated or unsaturated alicyclic-aliphatic hydrocarbon group, an aromatic hydrocarbon group, an aromatic-saturated or unsaturated alicyclic hydrocarbon group and the like can be mentioned, with preference given to those having 1 to 16, more preferably 1 to 6, carbon atoms. Specific examples thereof include alkyl group, alkenyl group, alkynyl group, cycloalkyl group, cycloalkenyl group, cycloalkylalkyl group, cycloalkenylalkyl group, aryl group and arylalkyl group and the like.

For example, the "alkyl group" is preferably a lower alkyl group (C₁₋₆ alkyl group) and the like, and, for example, a C₁₋₆ alkyl group such as methyl, ethyl, propyl, isopropyl, butyl, isobutyl, sec-butyl, tert-butyl, pentyl, 1-ethylpropyl, hexyl etc., and the like are generally used. For R, a lower alkyl group (C₁₋₆ alkyl group) is preferable, particularly a methyl group is preferable.

For example, the "alkenyl group" is preferably a lower alkenyl group and the like, and, for example, a C₂₋₇ alkenyl group such as vinyl, 1-propenyl, allyl, isopropenyl, butenyl, isobutenyl, 2,2-dimethyl-pent-4-enyl etc., and the like are generally used.

For example, the "alkynyl group" is preferably a lower alkynyl group and the like, and, for example, a C₂₋₆ alkynyl group such as ethynyl, propargyl, 1-propynyl etc., and the like are generally used.

For example, the "cycloalkyl group" is preferably a lower cycloalkyl group and the like, and, for example, a C₃₋₁₀ cycloalkyl group such as cyclopropyl, cyclobutyl, cyclopentyl, cyclohexyl, cycloheptyl, cyclooctyl, bicyclo[2.2.1]heptanyl and adamantyl etc., and the like are generally used.

For example, the "cycloalkenyl group" is preferably a lower cycloalkenyl group, and, for example, a C₃₋₁₀ cycloalkenyl group such as cyclopropenyl, cyclobutenyl, cyclopentenyl, cyclohexenyl, cycloheptenyl, cyclooctenyl, bicyclo[2.2.1]hept-5-en-2-yl etc., and the like are generally used.

For example, the "cycloalkylalkyl group" is preferably a lower cycloalkylalkyl group, and, for example, a C₄₋₉ cycloalkylalkyl group such as cyclopropylmethyl, cyclopropylethyl, cyclobutylmethyl, cyclopentylmethyl, cyclohexylmethyl and cyclohexylethyl etc., and the like are generally used.

For example, the "cycloalkenylalkyl group" is preferably a lower cycloalkenylalkyl group, and, for example, C₄₋₉ cycloalkenylalkyl such as cyclopentenylmethyl, cyclohexenylmethyl, cyclohexenylethyl, cyclohexenylpropyl, cycloheptenylmethyl, cycloheptenylethyl and bicyclo[2.2.1]hept-5-en-2-ylmethyl etc., and the like are generally used.

For example, the "aryl group" is preferably a C₆₋₁₄ aryl group such as phenyl, 1-naphthyl, 2-naphthyl, biphenylyl, 2-anthryl etc., and the like, and, for example, phenyl group and the like are generally used.

The "arylalkyl group" contains, as the aryl moiety, the "aryl group" defined above, and as the alkyl moiety, the "alkyl group" defined above. Of these, for example, a C₆₋₁₄ aryl-C₁₋₆ alkyl group is preferable, and, for example, benzyl, phenethyl and the like are generally used.

As the substituent that the "hydrocarbon group" of the "hydrocarbon group optionally having substituent(s)" represented by the above-mentioned E, R, R', R₁ and Y may have, for example, a halogen atom (e.g., fluorine, chlorine, bromine, iodine etc.), a nitro group, a cyano group, a hydroxy group, a thiol group, a sulfo group, a sulphino group, a phosphono group, an optionally halogenated lower alkyl group (e.g., C₁₋₆ alkyl such as methyl, ethyl, propyl, isopropyl, butyl, isobutyl, sec-butyl, tert-butyl, pentyl, 1-ethylpropyl, hexyl and the like, a mono-, di- or tri-halogeno-C₁₋₆ alkyl group such as chloromethyl, dichloromethyl, trichloromethyl, fluoromethyl, difluoromethyl, trifluoromethyl, 2-bromoethyl, 2,2,2-trifluoroethyl, pentafluoroethyl, 3,3,3-trifluoropropyl, 4,4,4-trifluorobutyl, 5,5,5-trifluoropentyl, 6,6,6-trifluorohexyl etc., and the like), an oxo group, an amidino group, an imino group, an alkylenedioxy group (e.g., C₁₋₃ alkylenedioxy group such as methylenedioxy, ethylenedioxy etc., and the like), a lower alkoxy group (e.g., C₁₋₆ alkoxy group such as methoxy, ethoxy, propoxy, isopropoxy, butoxy, isobutoxy, pentyloxy, hexyloxy etc., and the like), an optionally halogenated lower alkoxy group (e.g., a mono-, di-or tri-halogeno-C₁₋₆ alkoxy group such as chloromethyloxy, dichloromethyloxy, trichloromethyloxy, fluoromethyloxy, difluoromethyloxy, trifluoromethyloxy, 2-bromoethyloxy, 2,2,2-trifluoroethyloxy, pentafluoroethyloxy, 3,3,3-trifluoropropyloxy, 4,4,4-trifluorobutyloxy, 5,5,5-trifluoropentyloxy, 6,6,6-trifluorohexyloxy etc., and the like), a lower alkylthio group (e.g., a C₁₋₆ alkylthio group such as methylthio, ethylthio, propylthio, isopropylthio, butylthio, isobutylthio, pentylthio, hexylthio etc., and the like), a carboxyl group, a lower alkanoyl group (e.g., formyl; a C₁₋₆ alkyl-carbonyl group such as acetyl, propionyl, butyryl, isobutyryl etc., and the like), a lower alkanoyloxy group (e.g., formyloxy; a C₁₋₆ alkyl-carbonyloxy group such as acetyloxy, propionyloxy, butyryloxy, isobutyryloxy etc., and the like), a lower alkoxycarbonyl group (e.g., a C₁₋₆ alkoxy-carbonyl group such as methoxycarbonyl, ethoxycarbonyl, propoxycarbonyl, butoxycarbonyl etc., and the like), aralkyloxycarbonyl group (e.g., a C₇₋₁₁ aralkyloxy-carbonyl group such as benzyloxycarbonyl etc., and the like), a thiocarbamoyl group, a lower alkylsulfinyl group (e.g., a C₁₋₆ alkylsulfinyl group such as methylsulfinyl, ethylsulfinyl etc., and the like), a lower alkylsulfonyl group (e.g., a C₁₋₆ alkylsulfonyl group such as methylsulfonyl, ethylsulfonyl etc., and the like), a sulfamoyl group, a mono-lower alkylsulfamoyl group (e.g., a mono-C₁₋₆ alkylsulfamoyl group such as methylsulfamoyl, ethylsulfamoyl etc., and the like), di-lower alkylsulfamoyl group (e.g., a di-C₁₋₆ alkylsulfamoyl group such as dimethylsulfamoyl, diethylsulfamoyl etc., and the like), an arylsulfamoyl group (e.g., a C₆₋₁₀ arylsulfamoyl group such as phenylsulfamoyl, naphthylsulfamoyl etc., and the like), an aryl group (e.g., a C₆₋₁₀ aryl group such as phenyl, naphthyl etc., and the like), an aryloxy group (e.g., a C₆₋₁₀ aryloxy group such as phenyloxy, naphthyloxy etc., and the like), an arylthio group (e.g., a C₆₋₁₀ arylthio group such as phenylthio, naphthylthio etc., and the like), an arylsulfinyl group (e.g., a C₆₋₁₀ arylsulfinyl group such as phenylsulfinyl, naphthylsulfinyl etc., and the like), an arylsulfonyl group (e.g., a C₆₋₁₀ arylsulfonyl group such as phenylsulfonyl, naphthylsulfonyl etc., and the like), an arylcarbonyl group (e.g., a C₆₋₁₀ aryl-carbonyl group such as benzoyl, naphthoyl etc., and the like), an arylcarbonyloxy group (e.g., a C₆₋₁₀ aryl-carbonyloxy group such as benzoyloxy, naphthoyloxy etc., and the like), an optionally halogenated lower alkylcarbonylamino group (e.g., an optionally halogenated C₁₋₆ alkyl-carbonylamino group such as acetylamino, trifluoroacetylamino etc., and the like), a carbamoyl group optionally having substituent(s) (e.g., a group of the formula -CONR₂R₃ wherein R₂ and R₃ are each a hydrogen atom, a hydrocarbon group optionally having substituent(s) or a heterocyclic group optionally having substituent(s) and in the formula -CONR₂R₃, R₂ and R₃ may form a ring together with the adjacent nitrogen atom), an amino group optionally having substituent(s) (e.g., a group of the formula -NR₂R₃ wherein R₂ and R₃ are as defined above and in the formula -NR₂R₃, R₂ and R₃ may form a ring together with the adjacent nitrogen atom), a ureido group optionally having substituent(s) (e.g., a group of the formula -NHCONR₂R₃ wherein R₂ and R₃ are as defined above and in the formula -NHCONR₂R₃, R₂ and R₃ may form a ring together with the adjacent nitrogen atom), a carboxamide group optionally having substituent(s) (e.g., a group of the formula -NR₂COR₃ wherein R₂ and R₃ are as defined above) , a sulfonamide group optionally having substituent(s) (e.g., a group of the formula -NR₂SO₂R₃ wherein R₂ and R₃ are as defined above), a heterocyclic group optionally having substituent(s) (as defined for R₂ and R₃) and the like are used.

As the "hydrocarbon group" of the "hydrocarbon group optionally having substituent(s)" for R₂ and R₃, for example, a lower alkyl group (e.g., alkyl group having 1 to 6 carbon atoms such as methyl, ethyl, propyl group and the like), a lower alkenyl group (e.g., alkenyl group having 2 to 6 carbon atoms such as vinyl, allyl group and the like), a lower alkynyl group (e.g., alkynyl group having 2 to 6 carbon atoms such as ethynyl, propargyl group and the like), a cycloalkyl group (e.g., cycloalkyl group having 3 to 8 carbon atoms such as cyclopropyl, cyclobutyl, cyclopentyl, cyclohexyl group and the like), a cycloalkenyl group (e.g., cycloalkenyl group having 3 to 8 carbon atoms such as cyclobutenyl, cyclopentenyl, cyclohexenyl group and the like), a cycloalkylalkyl group (e.g. , C₃-C₈ cycloalkyl - C₁-C₆ alkyl group, such as cyclopropylmethyl, cyclobutylmethyl, cyclopentylmethyl, cyclohexylmethyl group and the like), a cycloalkenylalkyl group (e.g., C₃-C₈ cycloalkenyl - C₁-C₆ alkyl group, such as cyclobutenylmethyl, cyclopentenylmethyl, cyclohexenylmethyl group and the like), an aryl group (e.g., aryl group having 6 to 14 carbon atoms such as phenyl, naphthyl group and the like), an arylalkyl group (e.g., C₆-C₁₄ aryl - C₁-C₆ alkyl group, such as benzyl, naphthylmethyl group and the like) and the like can be mentioned.

As the "heterocyclic group" of the "heterocyclic group optionally having substituent(s)" represented by R₂ and R₃, a 5- to 12-membered monocyclic or fused heterocyclic group containing 1 or 2 kinds of 1 to 4 hetero atoms selected from nitrogen atom, sulfur atom and oxygen atom such as pyridyl, pyrrolidinyl, piperazinyl, piperidinyl, 2-oxoazepinyl, furyl, decahydroisoquinolyl, quinolyl, indolyl, isoquinolyl, thienyl, imidazolyl, morpholinyl etc., and the like can be mentioned. As the substituent for the "hydrocarbon group optionally having substituent(s)" and "heterocyclic group optionally having substituent(s)" for R₂ and R₃, for example, a halogen atom (e.g., fluorine, chlorine, bromine, iodine etc.), a lower alkyl group (e.g., alkyl group having 1 to 6 carbon atoms such as methyl, ethyl, propyl group and the like), a lower alkenyl group (e.g., alkenyl group having 2 to 6 carbon atoms such as vinyl, allyl group and the like), a lower alkynyl group (e.g., alkynyl group having 2 to 6 carbon atoms such as ethynyl, propargyl group and the like), a cycloalkyl group (e.g., cycloalkyl group having 3 to 8 carbon atoms such as cyclopropyl, cyclobutyl, cyclopentyl, cyclohexyl group and the like), a lower alkoxy group (e.g., alkoxy group having 1 to 6 carbon atoms such as methoxy, ethoxy group and the like), a nitro group, a cyano group, a hydroxy group, a thiol group, a carboxyl group, a lower alkanoyl group (e.g., formyl; C₁₋₆ alkyl-carbonyl group, such as acetyl, propionyl, butyryl group and the like), a lower alkanoyloxy group (e.g., formyloxy; C₁₋₆ alkyl-carbonyloxy group, such as acetyloxy, propionyloxy group and the like), a lower alkoxycarbonyl group (e.g., C₁₋₆ alkoxy-carbonyl group, such as methoxycarbonyl, ethoxycarbonyl, propoxycarbonyl group and the like), an aralkyloxycarbonyl group (e.g., C₇₋₁₇ aralkyloxy-carbonyl group, such as benzyloxycarbonyl group and the like), an aryl group (e.g., C₆-₁₄ aryl group, such as phenyl, naphthyl group and the like), an aryloxy group (e.g., C₆₋₁₄ aryloxy group having, such as phenyloxy, naphthyloxy group and the like), an arylcarbonyl group (e.g., C₆₋₁₄ aryl-carbonyl group, such as benzoyl, naphthoyl group and the like), an arylcarbonyloxy group (e.g., C₆₋₁₄ aryl-carbonyloxy group, such as benzoyloxy, naphthoyloxy group and the like), a carbamoyl group optionally having substituent(s) (e.g., carbamoyl; carbamoyl group mono- or di-substituted by alkyl group having 1 to 6 carbon atoms such as methylcarbamoyl, dimethylcarbamoyl group etc., and the like), an amino group optionally having substituent(s) (e.g., amino; amino group mono- or di-substituted by alkyl group having 1 to 6 carbon atoms such as methylamino, dimethylamino, ethylamino, diethylamino group etc., and the like) and the like can be mentioned. The number and the position of the substitutions are not particularly limited.

As the ring formed by R₂ and R₃ together with the adjacent nitrogen atom, for example, pyrrolidine, piperidine, homopiperidine, morpholine, piperazine, tetrahydroquinoline, tetrahydroisoquinoline and the like can be mentioned.

The "hydrocarbon group" of the "hydrocarbon group optionally having substituent(s)" represented by the above-mentioned E, R, R', R₁ and Y may have 1 to 5, preferably 1 to 3, the aforementioned substituent at substitutable positions of the hydrocarbon group, wherein, when the number of substituents is not less than 2, each substituent is the same or different.

As the "heterocyclic group" of the "heterocyclic group optionally having substituent(s)" represented by the above-mentioned E, R, R' and Y, a 5- to 12-membered aromatic heterocyclic group and saturated or unsaturated non-aromatic heterocyclic group containing, as ring-constituting atom (ring atom), 1 to 3 (preferably 1 or 2) kinds of at least 1 (preferably 1 to 4, more preferably 1 to 3) hetero atoms selected from oxygen atom, sulfur atom and nitrogen atom and the like can be mentioned. As the mentioned above, as the "heterocyclic group" of the "heterocyclic group optionally having substituent(s)" represented by Y, a saturated oxygen-containing heterocyclic group containing, as ring atoms, 1 to 4, more preferably 1 to 3, hetero atoms selected from oxygen atom, sulfur atom and nitrogen atom etc., and the like are preferable, particularly a 5- to 12-membered saturated oxygen-containing heterocyclic group and the like are preferable.

As the "aromatic heterocyclic group", an aromatic monocyclic heterocyclic group, an aromatic fused heterocyclic group and the like can be mentioned.

As the "aromatic monocyclic heterocyclic group", for example, a 5- or 6-membered aromatic monocyclic heterocyclic group such as furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl etc., and the like can be mentioned.

As the "aromatic fused heterocyclic group", for example, a 8- to 12-membered aromatic fused heterocyclic group (preferably a heterocyclic group wherein the aforementioned 5-or 6-membered aromatic monocyclic heterocyclic group is condensed with a benzene ring, or a heterocyclic group wherein the same or two different heterocyclic groups of the aforementioned 5- or 6-membered aromatic monocyclic heterocyclic group are condensed), such as benzofuranyl, isobenzofuranyl, benzothienyl, isobenzothienyl, indolyl, isoindolyl, 1H-indazolyl, benzimidazolyl, benzoxazolyl, 1,2-benzoisoxazolyl, benzothiazolyl, 1,2-benzoisothiazolyl, 1H-benzotriazolyl, quinolyl, isoquinolyl, cinnolinyl, quinazolinyl, quinoxalinyl, phthalazinyl, naphthylidinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl etc., and the like can be mentioned.

As the "saturated or unsaturated non-aromatic heterocyclic group", for example, a 3- to 8-membered (preferably 5- or 6-membered) saturated or unsaturated (preferably saturated) non-aromatic heterocyclic group (aliphatic heterocyclic group) such as oxiranyl, azetidinyl, oxetanyl, thietanyl, pyrrolidinyl, tetrahydrofuryl, thiolanyl, piperidinyl, tetrahydropyranyl, thianyl, morpholinyl, thiomorpholinyl, piperazinyl, azepanyl, oxepanyl, thiepanyl, oxazepanyl, thiazepanyl, azocanyl, oxocanyl, thiocanyl, oxazocanyl, thiazocanyl and the like can be mentioned. These may be oxo-substituted and examples thereof include 2-oxoazetidinyl, 2-oxopyrrolidinyl, 2-oxopiperidinyl, 2-oxoazepanyl, 2-oxoazocanyl, 2-oxotetrahydrofuryl, 2-oxotetrahydropyranyl, 2-oxothiolanyl, 2-oxothianyl, 2-oxopiperazinyl, 2-oxooxepanyl, 2-oxooxazepanyl, 2-oxothiepanyl, 2-oxothiazepanyl, 2-oxooxocanyl, 2-oxothiocanyl, 2-oxooxazocanyl, 2-oxothiazocanyl and the like. A 5-membered non-aromatic heterocyclic group such as 2-oxopyrrolidinyl and the like is preferable.

As the substituent that the "heterocyclic group" of the "heterocyclic group optionally having substituent(s)" represented by the above-mentioned E, R, R' and Y may have, for example, those similar to the "substituent" of the "hydrocarbon group optionally having substituent(s)" represented by the aforementioned E, R, R', R₁ and Y and the like are used.

The "heterocyclic group" of the "heterocyclic group optionally having substituent(s)" represented by E, R, R' and Y may each have 1 to 5, preferably 1 to 3, substituents mentioned above at substitutable positions of the heterocyclic group, and when the number of substituents is two or more, the substituents are the same or different.

The bond between R and W in the compound of the present invention is explained below. When R and W are bonded, the position of the bond between R and W is not particularly limited as long as R and W can be bonded. The same applies when R' and W' are bonded.

The bondable position of R and R' is the position where the "hydrocarbon group" and "substituent" of the "hydrocarbon group optionally having substituent(s)" defined above for R and R' can be bonded, and the position where the "heterocyclic group" and "substituent" of the "heterocyclic group optionally having substituent(s)" defined above for R and R' can be bonded.

As the bondable position of W and W', a bondable position of the "chain divalent hydrocarbon group" of the "chain divalent hydrocarbon group optionally having substituent(s)" defined above for W and W', a bondable position of the "chain divalent hydrocarbon group" defined above for W₁, W₁', W₂ and W₂', a bondable position of the "hydrocarbon ring" of the "hydrocarbon ring optionally having substituent(s)" defined above for ring Z, and a bondable position of the "heterocyclic group" of the "heterocyclic group optionally having substituent(s)" defined above for ring Z can be mentioned.

R and W, and R' and W' can be bonded at the bondable position thereof and can form a ring together with the adjacent nitrogen atom. As such ring, for example, a saturated nitrogen-containing ring (e.g., azetidine, pyrrolidine, piperidine, homopiperidine etc.), an unsaturated nitrogen-containing ring (e.g., tetrahydropyridine etc.), an aromatic nitrogen-containing ring (e.g., pyrrole etc.), a hetero ring (e.g., piperazine, morpholine etc.) containing, besides the nitrogen atom to which R and W are adjacent, at least one hetero atom selected from the group consisting of nitrogen, oxygen and sulfur, a fused ring (e.g., indole, indoline, isoindole, isoindoline, tetrahydroquinoline, tetrahydroisoquinoline etc.) and the like can be mentioned. Of these, a 4- to 7-membered ring is preferable.

The ring formed by R and W, or R' and W', which are bonded at each bondable position thereof, together with the adjacent nitrogen atom may have 1 to 4 substituents at substitutable positions thereof. When the number of substituents is 2 or more, the substituents are the same or different. As the substituent, the substituents of the "hydrocarbon group optionally having substituent(s)" and "heterocyclic group optionally having substituent(s)" defined for R and R', and the substituents of the "chain divalent hydrocarbon group optionally having substituent(s)" defined for W and W' can be mentioned. Specifically, a halogen atom (e.g., fluorine, chlorine, bromine, iodine etc.), a C₁₋₆ alkyl group such as methyl, ethyl, propyl, isopropyl, butyl, isobutyl, sec-butyl, tert-butyl, pentyl, 1-ethylpropyl, hexyl etc., and the like can be mentioned.

By the bond between R and W, or R' and W', for example, 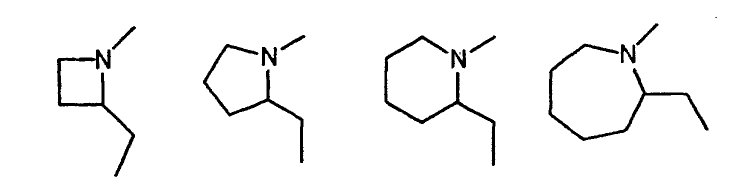 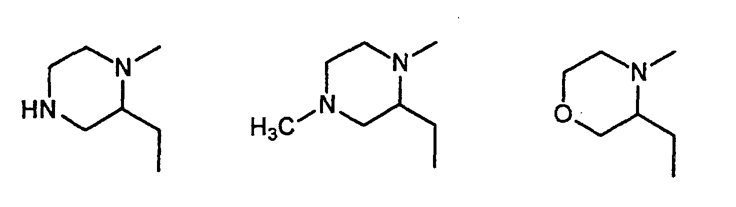 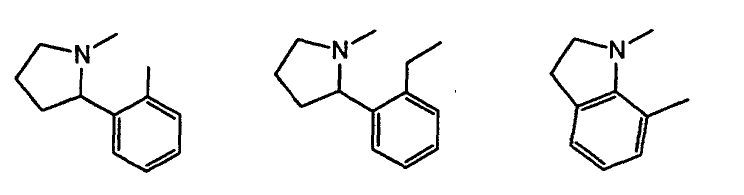 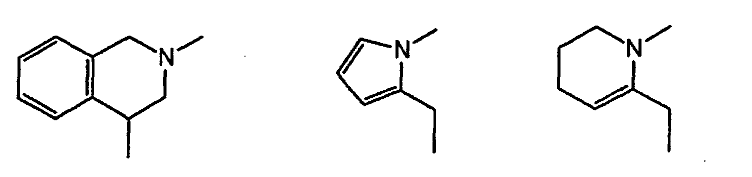 and the like are formed, but the ring is not limited to these. These may have substituents as defined above, and it would be understood for those of ordinary skill in the art that they may also have an isomer.

In the present invention, X represents a leaving group, such as a halogen atom, a benzotriazolyl group, a (2,5-dioxypyrrolidin-1-yl)oxy group and the like. Of these, a halogen atom such as fluorine, chlorine, bromine, iodine and the like is preferable, and chlorine is particularly preferable.

In the present invention, the "metal cation" is exemplified by alkali metal ion (e.g., Na⁺, K⁺, Li⁺, Cs⁺ and the like), with preference given to Na⁺.

In the present invention, the "quaternary ammonium ion" is exemplified by tetramethylammonium ion, tetraethylammonium ion, tetrapropylammonium ion, tetrabutylammonium ion and the like, with preference given to tetrabutylammonium ion.

In the prodrug compound (hereinafter compound (I), compound (II), compound (VII) and the like are to be referred to as a prodrug compound), a pharmacologically acceptable basic salt can be formed between an acidic group in a molecule and an inorganic base or an organic base etc, and a pharmacologically acceptable acid addition salt can be formed between a basic group in a molecule and an inorganic acid or an organic acid etc.

Examples of the inorganic basic salt of a prodrug compound of the present invention such as compound (I) and the like include salt with alkali metal (e.g., sodium, potassium and the like), alkaline earth metal (e.g., calcium and the like), ammonia etc., and the like, and examples of the organic basic salt of a prodrug compound of the present invention such as compound (I) and the like include salt with dimethylamine, triethylamine, piperazine, pyrrolidine, piperidine, 2-phenylethylamine, benzylamine, ethanolamine, diethanolamine, pyridine, collidine etc., and the like.

Examples of the acid addition salt of a prodrug compound of the present invention such as compound (I) and the like include inorganic acid salt (e.g., hydrochloride, sulfate, hydrobromide, phosphate and the like), organic acid salt (e.g., acetate, trifluoroacetate, succinate, maleate, fumarate, propionate, citrate, tartrate, lactate, oxalate, methanesulfonate, p-toluenesulfonate and the like) and the like.

When the prodrug compound of the present invention such as compound (I) and the like encompasses hydrates, examples of the "hydrate" include 0.5 hydrate - 5.0 hydrate. Of these, 0.5 hydrate, 1.0 hydrate, 1.5 hydrate and 2.0 hydrate are preferable.

The parent compound H-A (i.e., a compound obtained by eliminating the eliminatable modification group from compound (I) and the like, which are prodrugs) of the prodrug compound of the present invention such as compound (I) and the like is not particularly limited as long as it is a therapeutic agent having a group capable of bonding to a carbon atom of a modification group (side chain) eliminatable from a prodrug, via a carbon-oxygen bond, a carbon-sulfur bond or a carbon-nitrogen bond. The prodrug of the present application is specifically an optionally fused, nitrogen-containing heterocyclic group optionally having substituent(s), which is a compound represented by the formula (II): 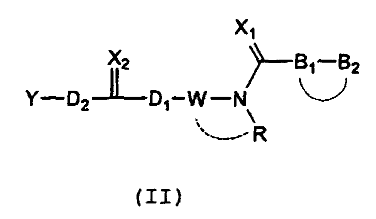 wherein -B₁-B₂ is a group remaining from elimination of hydrogen from a pharmaceutical compound H-B₁-B₂ wherein H-B₁- is a hydroxyl group, a thiol group, an amide group or an optionally fused, nitrogen-containing heterocycle optionally having substituent(s), which is capable of bonding to a carbon atom of a modification group (side chain) eliminatable from a prodrug, via a carbon-oxygen bond, a carbon-sulfur bond or a carbon-nitrogen bond, and other symbols are as defined above, or a salt thereof. Preferably, a compound wherein B₁ is a nitrogen-containing heterocyclic group optionally having substituent(s), which is capable of bonding to a carbon atom of a modification group (side chain) eliminatable from a prodrug, via a carbon-nitrogen bond, and which has an optionally fused ring, more preferably, a compound wherein the nitrogen-containing heterocyclic group represented by B₁ is a 5 or 6-membered aromatic heterocyclic group containing 1 to 4 nitrogens, and still more preferably, a compound wherein the nitrogen-containing aromatic heterocycle of the nitrogen-containing heterocyclic group represented by B₁ is imidazole, pyrrole, pyrazole, isoxazole, oxazole, thiazole or triazole.

As more preferable specific compounds, for example, the following compounds can be mentioned.

That is,
a compound represented by the formula (VII): 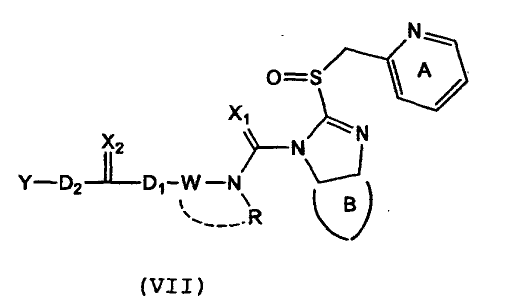 wherein ring A is an a pyridine ring optionally having substituent(s), ring B is a benzene ring optionally having substituent(s) or an aromatic monocyclic heterocycle optionally having substituent(s), and other symbols are as defined above, provided that R is not a hydrogen atom, or a salt thereof; and as a parent compound, a compound wherein an eliminatable modification group represented by the formula: 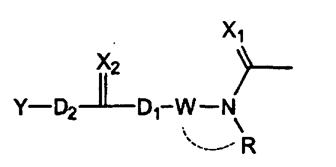 wherein each symbol is as defined above is introduced into a pharmaceutical compound such as Delavirdine, Rizatriptan, Dolasetron, Mepindolol, Lisuride, Tadalafil, Tropisetron, Sumatriptan, Frovatriptan, Terguride, Eletriptan, Zolmitriptan, Pergolide, Calvedidol, Bopindolol, Indoramin, Cabergoline, Almotriptan, Oxypertine, Procaterol, Dideoxyinosine, Zidovudine, Eperuvdine, Cimetidine, Dexmedetomidine, Pantoprazole, Ramosetron, Alosetron, Idarubicin, Epervudine, Cinolazepam, Befunolol, Pipotiazine, Opipramol, Pentostatin, Capecitabine, Prednicarbate, Enprostil, Halometasone, Bromperidol, Halofantrine, Penbutolol, Cladribine, Tramadol, Floxuridine, Galantamine, Acyclovir, Nadolol, Scopolamin, Ribavirin, Naftopidil, Dideoxycytidine, Hydroxydine, Haloperidol, Lormetazepam, Penciclovir and the like, and the like can be mentioned.

In the formula (VII), ring A is a "pyridine ring optionally having substituent(s)".

The pyridine ring of a "pyridine ring optionally having substituent(s)" for A ring optionally has 1 to 4 substituents. As the substituent, for example, a halogen atom (e.g., fluorine, chlorine, bromine, iodine etc.), a hydrocarbon group optionally having substituent(s) (e.g., alkyl group having 1 to 6 carbon atoms such as methyl group, ethyl group, n-propyl group etc. and the like), an amino group optionally having substituent(s) (e.g., amino; amino group mono-substituted or di-substituted by an alkyl group having 1 to 6 carbon atoms such as methylamino, dimethylamino, ethylamino, diethylamino group etc. and the like), an amide group (e.g., formamide, acetamide and the like C₁₋₃ acylamino group etc.), a lower alkoxy group optionally having substituent(s) (e.g., alkoxy group having 1 to 6 carbon atoms such as methoxy, ethoxy, 2,2,2-trifluoroethoxy, 3-methoxypropoxy group etc. and the like), a lower alkylenedioxy group (e.g., C₁₋₃ alkylenedioxy group such as methylenedioxy, ethylenedioxy etc. and the like) and the like can be mentioned.

As the substituent that the substituent of the "pyridine ring optionally having substituent(s)" for ring A can have, for example, a halogen atom (e.g., fluorine, chlorine, bromine, iodine etc.), a lower alkyl group (e.g., alkyl group having 1 to 6 carbon atoms such as methyl, ethyl, propyl group etc., and the like), a lower alkenyl group (e.g., alkenyl group having 2 to 6 carbon atoms such as vinyl, allyl group etc., and the like), a lower alkynyl group (e.g., alkynyl group having 2 to 6 carbon atoms such as ethynyl, propargyl group etc., and the like), a cycloalkyl group (e.g., cycloalkyl group having 3 to 8 carbon atoms such as cyclopropyl, cyclobutyl, cyclopentyl, cyclohexyl group etc., and the like), a lower alkoxy group (e.g., alkoxy group having 1 to 6 carbon atoms, such as methoxy, ethoxy group etc., and the like), a nitro group, a cyano group, a hydroxy group, a thiol group, a carboxyl group, a lower alkanoyl group (e.g., formyl; alkyl-carbonyl group having 1 to 6 carbon atoms such as acetyl, propionyl, butyryl group etc., and the like), a lower alkanoyloxy group (e.g., formyloxy; alkyl-carbonyloxy group having 1 to 6 carbon atoms such as acetyloxy, propionyloxy group etc., and the like), a lower alkoxycarbonyl group (e.g., alkoxy-carbonyl group having 1 to 6 carbon atoms such as methoxycarbonyl, ethoxycarbonyl, propoxycarbonyl group etc., and the like), an aralkyloxycarbonyl group (e.g., aralkyloxy-carbonyl group having 7 to 11 carbon atoms such as benzyloxycarbonyl group etc., and the like), an aryl group (e.g., aryl group having 6 to 14 carbon atoms such as phenyl, naphthyl group etc., and the like), an aryloxy group (e.g., aryloxy group having 6 to 14 carbon atoms such as phenyloxy, naphthyloxy group etc., and the like), an arylcarbonyl group (e.g., aryl-carbonyl group having 6 to 14 carbon atoms such as benzoyl, naphthoyl group etc., and the like), an arylcarbonyloxy group (e.g., aryl-carbonyloxy group having 6 to 14 carbon atoms such as benzoyloxy, naphthoyloxy group etc., and the like), a carbamoyl group optionally having substituent(s) (e.g., carbamoyl; carbamoyl group mono- or di-substituted by alkyl group having 1 to 6 carbon atoms, such as methylcarbamoyl, dimethylcarbamoyl group etc., and the like), an amino group optionally having substituent(s) (e.g., amino; amino group mono- or di-substituted by alkyl group having 1 to 6 carbon atoms, such as methylamino, dimethylamino, ethylamino, diethylamino group etc., and the like), and the like can be mentioned, wherein the number of substituents and the substitutable positions are not particularly limited.

While the number of substituents and the substitutable positions of the substituents of the "pyridine ring optionally having substituent(s)" for ring A are not particularly limited, 1 to 3 above-mentioned substituents are preferably substituted at any of the 3-, 4- and 5-positions of a pyridine ring.

As the "pyridine ring optionally having substituent(s)" for ring A, 3-methyl-4-(2,2,2-trifluoroethoxy)-2-pyridyl is preferable.

In the formula (VII), ring B shows "a benzene ring optionally having substituent (5) or "an aromatic monocyclic heterocycle optionally having substituent(s)", which is condensed with an imidazole moiety, with preference given to the former.

The benzene ring of the "benzene ring optionally having substituent(s)" for ring B optionally has 1 to 4 substituents at substitutable positions, and as the substituent, for example, a halogen atom (e.g., fluorine, chlorine, bromine, iodine etc.), a hydrocarbon group optionally having substituent(s) (e.g., alkyl group having 1 to 6 carbon atoms such as methyl group, ethyl group, n-propyl group etc., and the like), an amino group optionally having substituent(s) (e.g., amino; amino group mono- or di-substituted by alkyl group having 1 to 6 carbon atoms, such as methylamino, dimethylamino, ethylamino, diethylamino group and the like etc.), an amide group (e.g., C₁₋₃ acylamino group such as formamide, acetamide and the like etc.), a lower alkoxy group optionally having substituent(s) (e.g., alkoxy group having 1 to 6 carbon atoms such as methoxy, ethoxy, difluoromethoxy group and the like etc.), a lower alkylenedioxy group (e.g., C₁₋₃ alkylenedioxy group such as methylenedioxy, ethylenedioxy and the like etc.), and the like can be mentioned.

As the substituent that the substituent of the "benzene ring optionally having substituent(s)" for ring B can have, for example, a halogen atom (e.g., fluorine, chlorine, bromine, iodine etc.), a lower alkyl group (e.g., alkyl group having 1 to 6 carbon atoms such as methyl, ethyl, propyl group and the like etc.), a lower alkenyl group (e.g., alkenyl group having 2 to 6 carbon atoms such as vinyl, allyl group and the like etc.), a lower alkynyl group (e.g., alkynyl group having 2 to 6 carbon atoms such as ethynyl, propargyl group and the like etc.), a cycloalkyl group (e.g., cycloalkyl group having 3 to 8 carbon atoms such as cyclopropyl, cyclobutyl, cyclopentyl, cyclohexyl group and the like etc.), a lower alkoxy group (e.g., alkoxy group having 1 to 6 carbon atoms such as methoxy, ethoxy group and the like etc.), a nitro group, a cyano group, a hydroxy group, a thiol group, a carboxyl group, a lower alkanoyl group (e.g., formyl; alkyl-carbonyl group having 1 to 6 carbon atoms such as acetyl, propionyl, butyryl group and the like etc.), a lower alkanoyloxy group (e.g., formyloxy; alkyl-carbonyloxy group having 1 to 6 carbon atoms such as acetyloxy, propionyloxy group and the like etc.), a lower alkoxycarbonyl group (e.g., alkoxy-carbonyl group having 1 to 6 carbon atoms such as methoxycarbonyl, ethoxycarbonyl, propoxycarbonyl group and the like etc.), an aralkyloxycarbonyl group (e.g., aralkyloxy-carbonyl group having 7 to 17 carbon atoms such as benzyloxycarbonyl group and the like etc.), an aryl group (e.g., aryl group having 6 to 14 carbon atoms such as phenyl, naphthyl group etc., and the like), an aryloxy group (e.g., aryloxy group having 6 to 14 carbon atoms such as phenyloxy, naphthyloxy group and the like etc.), an arylcarbonyl group (e.g., aryl-carbonyl group having 6 to 14 carbon atoms such as benzoyl, naphthoyl group and the like etc.), an arylcarbonyloxy group (e.g., aryl-carbonyloxy group having 6 to 14 carbon atoms such as benzoyloxy, naphthoyloxy group and the like etc.), a carbamoyl group optionally having substituent(s) (e.g., carbamoyl; carbamoyl group mono- or di-substituted by alkyl group having 1 to 6 carbon atoms, such as methylcarbamoyl, dimethylcarbamoyl group and the like etc.), an amino group optionally having substituent(s) (e.g., amino; amino group mono- or di-substituted by alkyl group having 1 to 6 carbon atoms, such as methylamino, dimethylamino, ethylamino, diethylamino group and the like etc.), and the like can be mentioned, wherein the number of substituents and the substitutable positions are not particularly limited.

As the "benzene ring optionally having substituent(s)" for ring B, benzene ring is preferable.

As the "aromatic monocyclic heterocycle" of the "aromatic monocyclic heterocycle optionally having substituent(s) " for ring B, for example, a 5 or 6-membered aromatic monocyclic heterocycle, such as furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazan, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine and the like, and the like can be mentioned. These "aromatic monocyclic heterocycle" for ring B, among others, pyridine ring is preferable. They may have 1 to 4 substituents similar to those of the "benzene ring optionally having substituent(s)" at substitutable positions.

The position of condensation between the "aromatic monocyclic heterocycle" of the "aromatic monocyclic heterocycle optionally having substituent(s)" and an imidazole moiety is not particularly limited.

The present invention is not limited to the prodrug exemplified above. Particularly, the present invention can be preferably applied to a pharmaceutical compound having a nitrogen-containing heterocycle, particularly a nitrogen-containing heterocycle, which is a fused ring, especially a pharmaceutical compound having a nitrogen-containing aromatic heterocycle. Moreover, by introducing a modification group (side chain) eliminatable from the prodrug of the present invention into a nitrogen atom of the ring of a compound having imidazole, pyrazole, pyrrole, isoxazole, oxazole, thiazole or triazole, more preferable application can be achieved, because rapid expression of efficacy, prolongation of the efficacy, improvement of the chemical stability and the like can be afforded. As the above-mentioned compound (VII), a compound having a hydrocarbon ring group optionally having substituent(s) or a heterocyclic group optionally having substituent(s) for Z, a compound wherein X₁ and X₂ are oxygen atoms, a compound wherein D₁ and D₂ are each a bond or an oxygen atom (provided that D₁ and D₂ are not bonds at the same time), a compound wherein W is a chain divalent hydrocarbon group optionally having substituent(s), a compound wherein R is a C₁₋₆ hydrocarbon group optionally having substituent(s) (particularly lower alkyl group), and a compound wherein Y is a C₁₋₆ hydrocarbon group optionally having substituent(s), or a saturated heterocyclic group optionally having substituent(s), which contains, as a ring constituting atom, 1 to 4 hetero atoms selected from an oxygen atom, a nitrogen atom and a sulfur atom are preferable.

Particularly, a compound wherein X₁ and X₂ are oxygen atoms, D₁ and D₂ are each a bond or an oxygen atom (provided that D₁ and D₂ are not bonds at the same time), W is an ethylene group, R is a C₁₋₆ alkyl group, and Y is a saturated oxygen-containing heterocyclic group optionally having substituent(s), which further contains, as a ring constituting atom, 1 to 3 hetero atoms selected from an oxygen atom, a nitrogen atom and a sulfur atom is preferable.

As the "nitrogen-containing heterocycle as a partial structure capable of bonding to a modification group (side chain) eliminatable from a prodrug" of compound (II), a nitrogen-containing aromatic heterocycle is particularly preferable. As a particularly preferable nitrogen-containing heterocycle of the "optionally fused, nitrogen-containing heterocyclic group optionally having substituent(s)" of compound (II), for example, 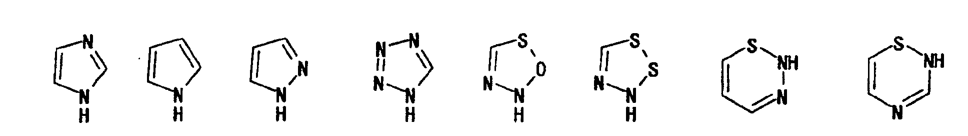 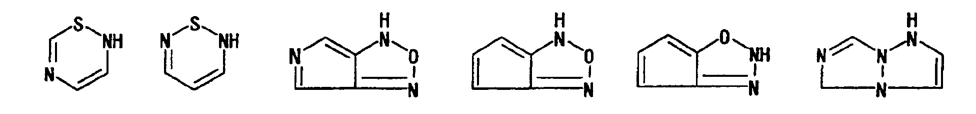 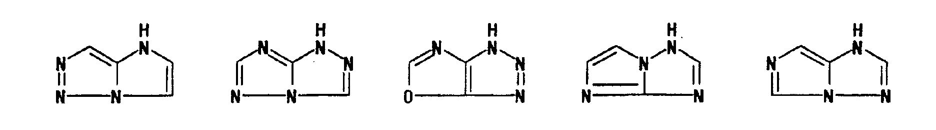 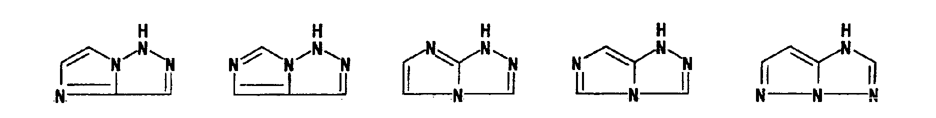 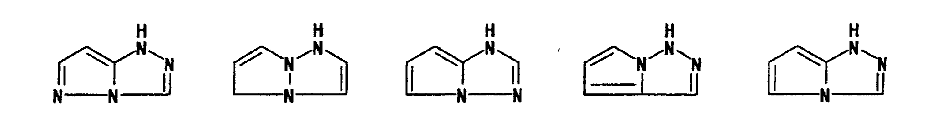 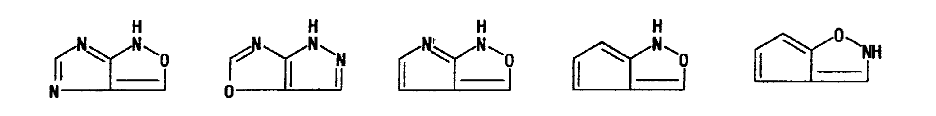 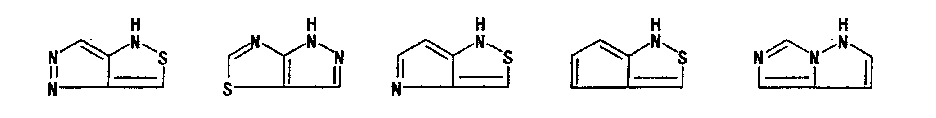 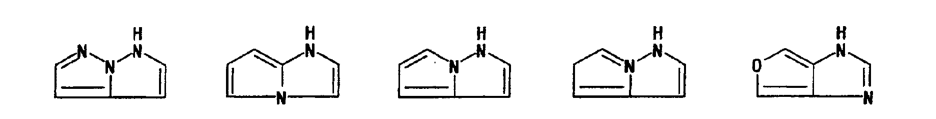 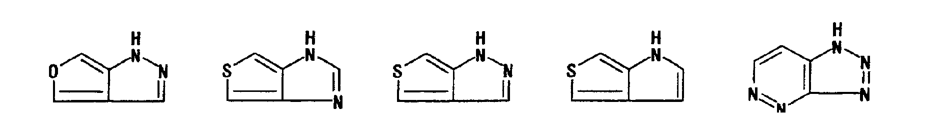 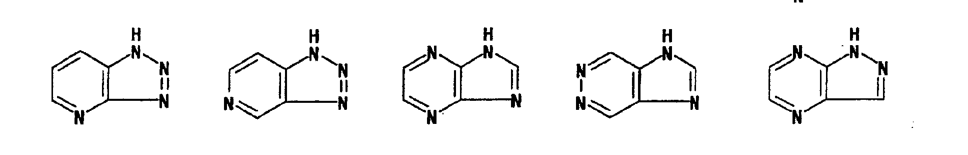 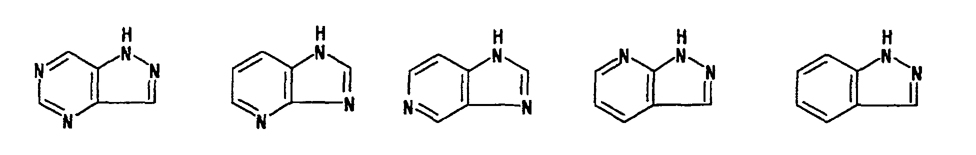 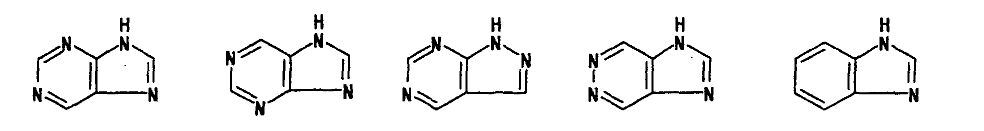 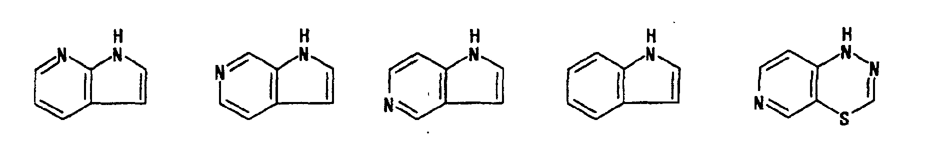 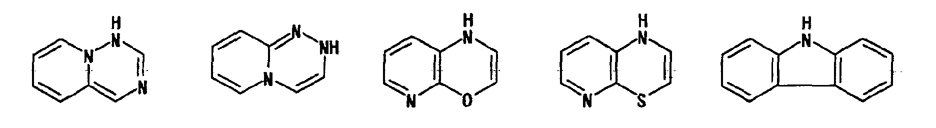 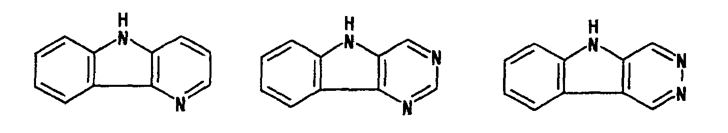 and the like can be mentioned.

As a particularly preferable nitrogen-containing aromatic heterocycle in the nitrogen-containing aromatic heterocyclic group included in the "optionally having substituent(s) nitrogen-containing heterocyclic group" of compound (II), for example, imidazole, pyrrole, pyrazole, isoxazole, oxazole, thiazole and triazole can be mentioned.

The prodrug compound of the present invention such as compound (I) and the like can be produced by the following Method A. 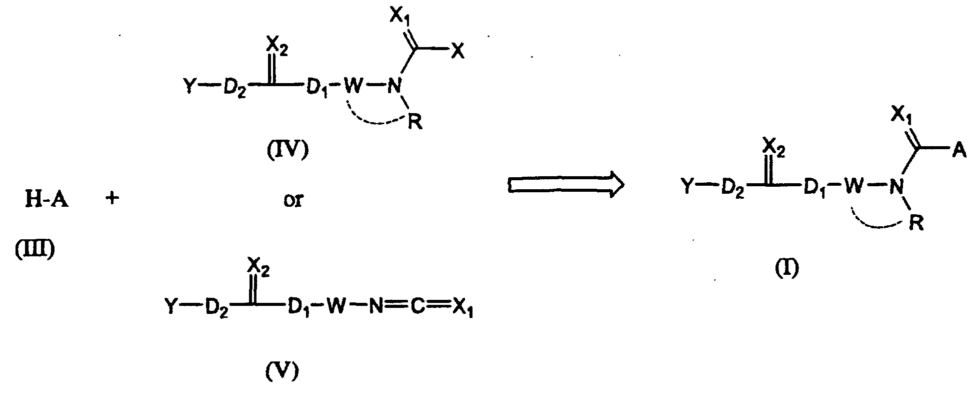 wherein each symbol in the formula is as defined above.

The prodrug compound of the present invention such as compound (I) and the like or a salt thereof can be obtained by condensation of a therapeutic drug or preventive drug H-A (III) having a partial structure capable of bonding to an eliminatable modifying group (side chain) of a prodrug, via a carbon-nitrogen bond, a carbon-sulfur bond or a carbon-oxygen bond, or a salt thereof, with compound (IV) or a salt thereof or compound (V) or a salt thereof, in the presence or absence of a base. The salts of compound (I), compound (III), compound (IV) and compound (V) are exemplified by, for example, acid addition salts such as inorganic acid salt (e.g., hydrochloride, sulfate, hydrobromide, phosphate and the like), organic acid salt (e.g., acetate, trifluoroacetate, succinate, maleate, fumarate, propionate, citrate, tartrate, lactate, oxalate, methanesulfonate, p-toluenesulfonate and the like), and the like can be mentioned.

The reaction of Method A is generally conducted in a solvent, and a solvent that does not inhibit the reaction of Method A is selected as appropriate. Examples of such solvent include ethers (e.g., dioxane, tetrahydrofuran, diethyl ether, tert-butyl methyl ether, diisopropyl ether, ethylene glycol dimethyl ether and the like), esters (e.g., ethyl formate, ethyl acetate, butyl acetate and the like), halogenated hydrocarbons (e.g., dichloromethane, chloroform, carbon tetrachloride, trichlene, 1,2-dichloroethane and the like), hydrocarbons (e.g., n-hexane, benzene, toluene and the like), amides (e.g., formamide, N,N-dimethylformamide, N,N-dimethylacetamide and the like), ketones (e.g., acetone, methyl ethyl ketone, methyl isobutyl ketone and the like), nitriles (e.g., acetonitrile, propionitrile and the like) and the like, as well as dimethyl sulfoxide, sulfolane, hexamethylphosphoramide, water and the like, which may be used alone or as a mixed solvent. The amount of the solvent to be used is not particularly limited as long as the reaction mixture can be stirred, which is generally 2- to 100-fold amount by weight, preferably 5- to 50-fold amount by weight, relative to 1 mole of compound (III) or a salt thereof.

The amount of compound (IV) or a salt thereof or compound (V) or a salt thereof to be used is generally 1 - 10 mol, preferably 1 - 3 mol, relative to 1 mol of compound (III) or a salt thereof.

The reaction of Method A is carried out within a temperature range of from about 0°C to 100°C, preferably 20°C to 80°C.

The reaction time of Method A varies depending on the kind of compounds (III), (IV), (V) or a salt thereof and solvent, reaction temperature and the like, but it is generally 1 min. - 96 hrs., preferably 1 min. - 72 hrs., more preferably 15 min. - 24 hrs.

The base in Method A is, for example, an inorganic base (e.g., sodium carbonate, potassium carbonate, calcium carbonate, sodium hydrogen carbonate etc.), a tertiary amine (e.g., triethylamine, tripropylamine, tributylamine, cyclohexyldimethylamine, pyridine, lutidine, γ-collidine, N,N-dimethylaniline, N-methylpiperidine, N-methylpyrrolidine, N-methylmorpholine, 4-dimethylaminopyridine and the like); alkylene oxides (e.g., propylene oxide, epichlorohydrin etc.) and the like. The amount of the base to be used is generally 0.01 mol - 10 mol, preferably 1 mol - 3 mol, relative to 1 mol of compound (III) or a salt thereof.

The compound (IV) and a salt thereof can be produced according to a method known per se or a method analogous thereto. For example, when X is a chlorine atom, it can be obtained by reacting a compound represented by the formula (VIII) : 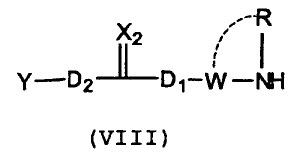 wherein each symbol is as defined above, or a salt thereof with phosgene, trichloromethyl chloroformate, bis(trichloromethyl)carbonate, thiophosgene and the like in the presence of an acid scavenger in a solvent (e.g., tetrahydrofuran, acetonitrile, dichloromethane etc.). Alternatively, compound (IV) can be also obtained by treating ethylcarbamate, which is obtained by reacting compound (VIII) or a salt thereof with ethyl chloroformate, with phosphorus oxychloride according to the method described in Synthetic Communications, vol. 17, p. 1887 (1987) or a method analogous thereto. As the salt of compound (VIII), for example, acid addition salts such as inorganic acid salts (e.g., hydrochloride, sulfate, hydrobromide, phosphate etc.), organic acid salts (e.g., acetate, trifluoroacetate, succinate, maleate, fumarate, propionate, citrate, tartrate, lactate, oxalate, methanesulfonate, p-toluenesulfonate etc.), and the like can be mentioned.

As the acid scavenger used here, for example, inorganic bases (e.g., sodium carbonate, potassium carbonate, calcium carbonate, sodium hydrogen carbonate etc.), tertiary amine (e.g., triethylamine, tripropylamine, tributylamine, cyclohexyldimethylamine, pyridine, lutidine, γ-collidine, N,N-dimethylaniline, N-methylpiperidine, N-methylpyrrolidine, N-methylmorpholine, 4-dimethylaminopyridine etc.) and the like can be mentioned.

The compound (VIII) and a salt thereof can be produced according to a method known per se or a method analogous thereto. For example, when D₁ is other than a bond, compound (VIII) can be obtained by condensing a compound represented by the formula (IX): 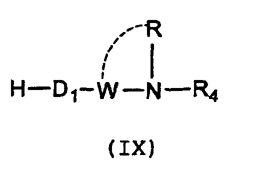 wherein R₄ is a hydrogen atom or nitrogen-protecting group, and other symbols are as defined above, or a salt thereof with carboxylic acid or thionic acid represented by the formula (X) : 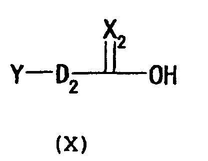 wherein each symbol is as defined above, or a reactive derivative thereof (e.g., anhydride, halide etc.), or a salt thereof in a suitable solvent (e.g., ethyl acetate, tetrahydrofuran, dichloromethane, N,N-dimethylformamide etc., followed by deprotection as necessary. As the salt of compound (IX), for example, acid addition salts such as inorganic acid salts (e.g., hydrochloride, sulfate, hydrobromide, phosphate etc.), organic acid salts (e.g., acetate, trifluoroacetate, succinate, maleate, fumarate, propionate, citrate, tartrate, lactate, oxalate, methanesulfonate, p-toluenesulfonate etc.) etc., and the like can be mentioned.

Alternatively, when D₁ is a bond, compound (VIII) can be obtained by condensing carboxylic acid or thionic acid represented by the formula (XI): 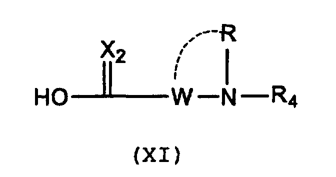 wherein each symbol is as defined above, or a reactive derivative thereof (e.g., anhydride, halide etc.), or a salt thereof with a compound represented by Y-D₂-H in a suitable solvent (e.g., ethyl acetate, tetrahydrofuran, dichloromethane, N,N-dimethylformamide etc.), followed by deprotection, as necessary. As the salt of compound (XI), for example, acid addition salts such as inorganic acid salts (e.g., hydrochloride, sulfate, hydrobromide, phosphate etc.), organic acid salts (e.g., acetate, trifluoroacetate, succinate, maleate, fumarate, propionate, citrate, tartrate, lactate, oxalate, methanesulfonate, p-toluenesulfonate etc.) and the like, salts with alkali metal (e.g., sodium, potassium etc.), alkaline earth metal (e.g., calcium etc.), ammonia etc., and the like, and for example, organic base such as dimethylamine, triethylamine, piperazine, pyrrolidine, piperidine, 2-phenylethylamine, benzylamine, ethanolamine, diethanolamine, pyridine, collidine etc., and the like can be mentioned.

As the protecting group represented by R₄ in the formulas (IX) and (XI), for example, a formyl group, a C₁₋₆ alkyl-carbonyl group (e.g., acetyl, ethylcarbonyl etc.), a benzyl group, a tert-butyloxycarbonyl group, a benzyloxycarbonyl group, an allyloxycarbonyl group, a C₇₋₁₀ aralkyl-carbonyl group (e.g., benzylcarbonyl etc.), a trityl group and the like are used. These groups may be substituted by 1 to 3 halogen atoms (e.g., fluorine, chlorine, bromine etc.), a nitro group and the like.

As a method for removing such protecting groups, a method known *per se* or a method analogous thereto is used, which is, for example, a method using an acid, a base, reduction, UV light, palladium acetate etc., and the like are used.

Compound (V) or a salt thereof can be produced by a method known per se or a method analogous thereto. For example, it can be obtained by reacting a compound represented by the formula (XII): 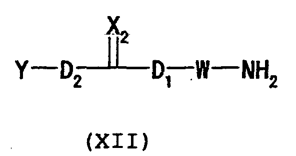 wherein each symbol is as defined above, or a salt thereof with phosgene, tri-chloromethyl chloroformate, bis(tri-chloromethyl)carbonate, thiophosgene and the like in the presence or absence of an acid scavenger in a solvent (e.g., tetrahydrofuran, 1,4-dioxane, acetonitrile, dichloromethane, 1,2-dichloroethane, dimethylformamide etc.). As the salt of compound (XII), for example, acid addition salts such as inorganic acid salts (e.g., hydrochloride, sulfate, hydrobromide, phosphate etc.), organic acid salts (e.g., acetate, trifluoroacetate, succinate, maleate, fumarate, propionate, citrate, tartrate, lactate, oxalate, methanesulfonate, p-toluenesulfonate etc.) and the like, and the like can be mentioned.

As the acid scavenger here, for example, inorganic bases (e.g., sodium carbonate, potassium carbonate, calcium carbonate, sodium hydrogen carbonate etc.), tertiary amines (e.g., triethylamine, tripropylamine, tributylamine, cyclohexyldimethylamine, pyridine, lutidine, γ-collidine, N,N-dimethylaniline, N-methylpiperidine, N-methylpyrrolidine, N-methylmorpholine, 4-dimethylaminopyridine etc.) and the like can be mentioned.

Compound (XII) or a salt thereof can be produced by a method known per se or a method analogous thereto. For example, when D₁ is other than a bond, it can be obtained by condensing a compound represented by the formula (XIII): 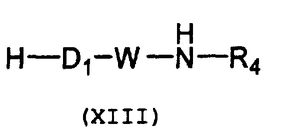 wherein R₄ is a hydrogen atom or a nitrogen-protecting group, and other symbols are as defined above, or a salt thereof, with carboxylic acid or thionic acid represented by the formula (X): 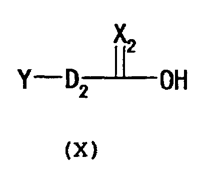 wherein each symbol is as defined above, or a reactive derivative thereof (e.g., anhydride, halide etc.), or a salt thereof, in a suitable solvent (e.g., ethyl acetate, tetrahydrofuran, dichloromethane, N,N-dimethylformamide etc.), and deprotection as necessary. As the salts of compound (XIII), for example, acid addition salts such as inorganic acid salts (e.g., hydrochloride, sulfate, hydrobromide, phosphate etc.), organic acid salts (e.g., acetate, trifluoroacetate, succinate, maleate, fumarate, propionate, citrate, tartrate, lactate, oxalate, methanesulfonate, p-toluenesulfonate etc.) and the like, and the like can be mentioned.

Alternatively, when D₁ is a bond, the compound can be obtained by condensing carboxylic acid or thionic acid represented by the formula (XIV): 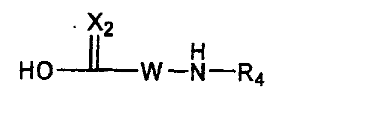 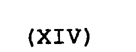 wherein each symbol is as defined above, or a reactive derivative thereof (e.g., anhydride, halide etc.), or a salt thereof, with a compound represented by Y-D₂-H in a suitable solvent (e.g., ethyl acetate, tetrahydrofuran, dichloromethane, N,N-dimethylformamide etc.), followed by deprotection as necessary. As the salts of compound (XIV), for example, acid addition salts such as inorganic acid salts (e.g., hydrochloride, sulfate, hydrobromide, phosphate etc.), organic acid salts (e.g., acetate, trifluoroacetate, succinate, maleate, fumarate, propionate, citrate, tartrate, lactate, oxalate, methanesulfonate, p-toluenesulfonate etc.) and the like, for example, salts with alkaline metal (e.g., sodium, potassium etc.), alkaline earth metal (e.g., calcium etc.), ammonia and the like, and the like, and, for example, organic basic salts with dimethylamine, triethylamine, piperazine, pyrrolidine, piperidine, 2-phenylethylamine, benzylamine, ethanolamine, diethanolamine, pyridine, collidine and the like, and the like can be mentioned.

As the protecting group represented by R₄, for example, a formyl group, a C₁₋₆ alkyl-carbonyl group (e.g., acetyl, ethylcarbonyl etc.), a benzyl group, a tert-butyloxycarbonyl group, a benzyloxycarbonyl group, an allyloxycarbonyl group, a C₇₋₁₀ aralkyl-carbonyl group (e.g., benzylcarbonyl etc.), a trityl group and the like are used. These groups may be substituted by 1 to 3 halogen atoms (e.g., fluorine, chlorine, bromine etc.), a nitro group and the like.

As an elimination method of these protecting groups, a method known per se or a method analogous thereto is used, and, for example, a method using acid, base, reduction, ultraviolet light, palladium acetate and the like, and the like are used.

Compound (VI) or a salt thereof can be produced by a method known per se or a method analogous thereto. For example, the compound can be obtained by reacting a compound represented by the formula (XV): 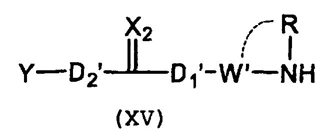 wherein each symbol is as defined above, or a salt thereof, with phosgene, trichloromethyl chloroformate, bis(tri-chloromethyl)carbonate, thiophosgene and the like, in the presence of an acid scavenger, in a solvent (e.g., tetrahydrofuran, acetonitrile, dichloromethane etc.). Alternatively, the compound can be also obtained by treating ethylcarbamate obtained by reacting compound (XV) or a salt thereof with chloroethyl formate with phosphorus oxychloride by a method described in Synthetic Communications, vol. 17, p. 1887 (1987) or a method analogous thereto. As the salts of compound (XV), for example, acid addition salts such as inorganic acid salts (e.g., hydrochloride, sulfate, hydrobromide, phosphate etc.), organic acid salts (e.g., acetate, trifluoroacetate, succinate, maleate, fumarate, propionate, citrate, tartrate, lactate, oxalate, methanesulfonate, p-toluenesulfonate etc.) and the like, and the like can be mentioned.

As the acid scavenger here, for example, inorganic bases (e.g., sodium carbonate, potassium carbonate, calcium carbonate, sodium hydrogen carbonate etc.), tertiary amines (e.g., triethylamine, tripropylamine, tributylamine, cyclohexyldimethylamine, pyridine, lutidine, γ-collidine, N,N-dimethylaniline, N-methylpiperidine, N-methylpyrrolidine, N-methylmorpholine, 4-dimethylaminopyridine etc.) and the like can be mentioned.

Compound (XV) or a salt thereof can be produced by a method known *per se* or a method analogous thereto. For example, when D₁' is other than a bond, the compound can be obtained by condensing a compound represented by the formula (XVI): 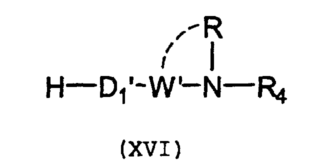 wherein R₄ is a hydrogen atom or a nitrogen-protecting group, and other symbols are as defined above, or a salt thereof with carboxylic acid or thionic acid represented by the formula (XVII): 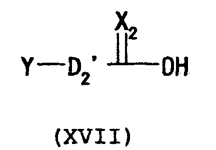 wherein each symbol is as defined above, or a reactive derivative thereof (e.g., anhydride, halide etc.), or a salt thereof, in a suitable solvent (e.g., ethyl acetate, tetrahydrofuran, dichloromethane, N,N-dimethylformamide etc.), followed by deprotection as necessary. As the salts of compound (XVII), for example, acid addition salts such as inorganic acid salts (e.g., hydrochloride, sulfate, hydrobromide, phosphate etc.), organic acid salts (e.g., acetate, trifluoroacetate, succinate, maleate, fumarate, propionate, citrate, tartrate, lactate, oxalate, methanesulfonate, p-toluenesulfonate etc.) and the like, and the like can be mentioned.

Alternatively, when D₁' is a bond, the compound can be obtained by condensing carboxylic acid or thionic acid represented by the formula (XVIII): 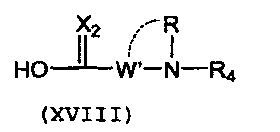 wherein each symbol is as defined above, or a reactive derivative thereof (e.g., anhydride, halide etc.), or a salt thereof, with a compound represented by Y-D₂'-H in a suitable solvent (e.g., ethyl acetate, tetrahydrofuran, dichloromethane, N,N-dimethylformamide etc.), followed by deprotection as necessary. As the salts of compound (XVIII), for example, acid addition salts such as inorganic acid salts (e.g., hydrochloride, sulfate, hydrobromide, phosphate etc.), organic acid salts (e.g., acetate, trifluoroacetate, succinate, maleate, fumarate, propionate, citrate, tartrate, lactate, oxalate, methanesulfonate, p-toluenesulfonate etc.) and the like, for example, salts with alkaline metal (e.g., sodium, potassium etc.), alkaline earth metal (e.g., calcium etc.), ammonia and the like, and, for example, organic basic salts with dimethylamine, triethylamine, piperazine, pyrrolidine, piperidine, 2-phenylethylamine, benzylamine, ethanolamine, diethanolamine, pyridine, collidine and the like, and the like can be mentioned.

As the protecting group for R₄, for example, a formyl group, a C₁₋₆ alkyl-carbonyl group (e.g., acetyl, ethylcarbonyl etc.), a benzyl group, a tert-butyloxycarbonyl group, a benzyloxycarbonyl group, an allyloxycarbonyl group, a C₇₋₁₀ aralkyl-carbonyl group (e.g., benzylcarbonyl etc.), a trityl group and the like are used. These groups may be substituted by 1 to 3 halogen atoms (e.g., fluorine, chlorine, bromine etc.), a nitro group and the like.

As an elimination method of these protecting groups, a method known per se or a method analogous thereto is used, and, for example, a method using acid, base, reduction, ultraviolet light, palladium acetate and the like, and the like are used.

The prodrug of the present invention can be used according to a pharmaceutical agent containing the parent compound. That is, as long as the parent compound is free of toxicity, a prodrug of the present invention, into which a eliminatable modification group is introduced, is free of toxicity and safe, and can be used for the prophylaxis or treatment by safely administering to mammals, including human, in a suitable dosage form according to the dose, subject of administration, administration route, target disease and the like, in the case of administration of the parent compound of the prodrug. When a pharmaceutical composition is to be produced, the content of the prodrug of the present invention, such as compound (I) and the like or a salt thereof, is about 0.01 to 100% by weight relative to the entire composition. Though subject to change depending on the administration target, administration route, disease and the like, when, for example, a representative compound (VII) contained in the prodrug compound of the present invention such as compound (I) and the like is used as an anti-ulcer agent, its dose to an adult is about 0.5 to 1,500 mg/day, preferably about 5 to 150 mg/day, based on the active ingredient, when, for example, the compound is orally administered to an adult human (60 kg). The prodrug compound of the present invention such as compound (I) or a salt thereof may be administered once daily or in 2 or 3 divided portions per day.

The pharmacologically acceptable carrier that may be used to produce a pharmaceutical composition containing the prodrug compound of the present invention includes various organic or inorganic carrier substances in common use as pharmaceutical materials, including excipients, lubricants, binders, disintegrants, water-soluble polymers and basic inorganic salts for solid preparations; and solvents, dissolution aids, suspending agents, isotonizing agents, buffers and soothing agents for liquid preparations and the like. Other ordinary pharmaceutical additives such as preservatives, anti-oxidants, coloring agents, sweetening agents, souring agents, bubbling agents and flavorings may also be used as necessary.

Such "excipients" include, for example, lactose, sucrose, D-mannitol, starch, cornstarch, crystalline cellulose, light silicic anhydride, titanium oxide and the like.

Such "lubricants" include, for example, magnesium stearate, sucrose fatty acid esters, polyethylene glycol, talc, stearic acid and the like.

Such "binders" include, for example, hydroxypropyl cellulose, hydroxypropylmethyl cellulose, crystalline cellulose, starch, polyvinylpyrrolidone, powdered acacia, gelatin, pullulan, low-substituted hydroxypropyl cellulose and the like.

Such "disintegrants" include (1) crosslinked povidone, (2) what is called super-disintegrants such as crosslinked carmellose sodium (FMC-Asahi Chemical) and carmellose calcium (Gotoku Yakuhin) etc, (3) carboxymethyl starch sodium (e.g., product of Matsutani Chemical), (4) low-substituted hydroxypropyl cellulose (e.g., product of Shin-Etsu Chemical), (5) cornstarch, and so forth. Said "crosslinked povidone" may be any crosslinked polymer having the chemical name 1-ethenyl-2-pyrrolidinone homopolymer, including polyvinylpyrrolidone (PVPP) and 1-vinyl-2-pyrrolidinone homopolymer, and is exemplified by Colidon CL (produced by BASF), Polyplasdon XL (produced by ISP), Polyplasdon XL-10 (produced by ISP), Polyplasdon INF-10 (produced by ISP) and the like.

Such "water-soluble polymers" include, for example, ethanol-soluble water-soluble polymers [e.g., cellulose derivatives such as hydroxypropyl cellulose (hereinafter also referred to as HPC) etc, polyvinylpyrrolidone and the like], ethanol-insoluble water-soluble polymers [e.g., cellulose derivatives such as hydroxypropylmethyl cellulose (hereinafter also referred to as HPMC) etc., methyl cellulose, carboxymethyl cellulose sodium and the like, sodium polyacrylate, polyvinyl alcohol, sodium alginate, guar gum and the like] and the like.

Such "basic inorganic salts" include, for example, basic inorganic salts of sodium, potassium, magnesium and/or calcium. Preferred are basic inorganic salts of magnesium and/or calcium. More preferred are basic inorganic salts of magnesium Such basic inorganic salts of sodium include, for example, sodium carbonate, sodium hydrogen carbonate, disodium hydrogen phosphate and the like. Such basic inorganic salts of potassium include, for example, potassium carbonate, potassium hydrogen carbonate and the like. Such basic inorganic salts of magnesium include, for example, heavy magnesium carbonate, magnesium carbonate, magnesium oxide, magnesium hydroxide, magnesium metasilicate aluminate, magnesium silicate, magnesium aluminate, synthetic hydrotalcite [Mg₆Al₂(OH)₁₆CO₃4H₂O], and alumina hydroxide magnesium. Preferred are heavy magnesium carbonate, magnesium carbonate, magnesium oxide, magnesium hydroxide and the like. Such basic inorganic salts of calcium include, for example, precipitated calcium carbonate, calcium hydroxide, etc.

Such "solvents" include, for example, water for injection, alcohol, propylene glycol, macrogol, sesame oil, corn oil, olive oil and the like.

Such "dissolution aids" include, for example, polyethylene glycol, propylene glycol, D-mannitol, benzyl benzoate, ethanol, trisaminomethane, cholesterol, triethanolamine, sodium carbonate, sodium citrate and the like.

Such "suspending agents" include, for example, surfactants such as stearyltriethanolamine, sodium lauryl sulfate, laurylaminopropionic acid, lecithin, benzalkonium chloride, benzethonium chloride, monostearic glycerol etc; hydrophilic polymers such as polyvinyl alcohol, polyvinylpyrrolidone, carboxymethyl cellulose sodium, methyl cellulose, hydroxymethyl cellulose, hydroxyethyl cellulose, hydroxypropyl cellulose etc., and the like.

Such "isotonizing agents" include, for example, glucose, D-sorbitol, sodium chloride, glycerol, D-mannitol and the like.

Such "buffers" include, for example, buffer solutions of phosphates, acetates, carbonates, citrates etc, and the like.

Such "soothing agents" include, for example, benzyl alcohol and the like.

Such "preservatives" include, for example, p-oxybenzoic acid esters, chlorobutanol, benzyl alcohol, phenethyl alcohol, dehydroacetic acid, sorbic acid and the like.

Such "antioxidants" include, for example, sulfites, ascorbic acid, α-tocopherol and the like.

Such "coloring agents" include, for example, food colors such as Food Color Yellow No. 5, Food Color Red No. 2, Food Color Blue No. 2 etc; food lake colors, red oxide and the like.

Such "sweetening agents" include, for example, saccharin sodium, dipotassium glycyrrhizinate, aspartame, stevia, thaumatin and the like.

Such "souring agents" include, for example, citric acid (citric anhydride), tartaric acid, malic acid and the like.

Such "bubbling agents" include, for example, sodium bicarbonate and the like.

Such "flavorings" may be synthetic substances or naturally occurring substances, and include, for example, lemon, lime, orange, menthol, strawberry and the like.

The prodrug compound of the present invention may be prepared as a preparation for oral administration in accordance with a method known *per se*, by, for example, compression-shaping in the presence of a carrier such as an excipient, a disintegrant, a binder, a lubricant, or the like, and subsequently coating the preparation as necessary by a method known per se for the purpose of taste masking, enteric dissolution or sustained release. For an enteric-coated preparation, an intermediate layer may be provided by a method known *per se* between the enteric layer and the pharmaceutical compound-containing layer for the purpose of separation of the two layers.

For preparing a prodrug such as the compound (I) and the like of the present invention as an orally disintegrating tablet, available methods include, for example, a method in which a core containing crystalline cellulose and lactose is coated with a prodrug such as the compound (I) and the like of the present invention and, where necessary, a basic inorganic salt, and then further coated with a coating layer containing a water-soluble polymer to give a composition, which is coated with an enteric coating layer containing polyethylene glycol, further coated with an enteric coating layer containing triethyl citrate, still further coated with an enteric coating layer containing polyethylene glycol, and finally coated with mannitol to give fine granules, which are mixed with additives and shaped.

The above-mentioned "enteric coating layer" includes, for example, a layer consisting of a mixture of one or more kinds from aqueous enteric polymer substrates such as cellulose acetate phthalate (CAP), hydroxypropylmethyl cellulose phthalate, hydroxymethyl cellulose acetate succinate, methacrylic acid copolymers (e.g., Eudragit L30D-55 (trade name; produced by Rohm), Colicoat MAE30DP (trade name; produced by BASF), Polyquid PA30 (trade name; produced by Sanyo Chemical) etc), carboxymethylethyl cellulose, shellac and the like; sustained-release substrates such as methacrylic acid copolymers (e.g., Eudragit NE30D (trade name), Eudragit RL30D (trade name), Eudragit RS30D (trade name), etc.) and the like; water-soluble polymers; plasticizers such as triethyl citrate, polyethylene glycol, acetylated monoglycerides, triacetin, castor oil etc.; and the like; and the like.

The above-mentioned "additive" includes, for example, water-soluble sugar alcohols (e.g., sorbitol, mannitol, maltitol, reduced starch saccharides, xylitol, reduced palatinose, erythritol, etc.), crystalline cellulose (e.g., Ceolas KG 801, Avicel PH 101, Avicel PH 102, Avicel PH 301, Avicel PH 302, Avicel RC-591 (crystalline cellulose carmellose sodium) etc), low-substituted hydroxypropyl cellulose (e.g., LH-22, LH-32, LH-23, LH-33 (Shin-Etsu Chemical), mixtures thereof etc) and the like. Furthermore, binders, souring agents, bubbling agents, sweetening agents, flavorings, lubricants, coloring agents, stabilizers, excipients, disintegrators etc. are also used.

The prodrug compound of the present invention may be used in combination with 1 to 3 other active ingredients for the purpose of enhancing the efficacy or allowing exertion of efficacy while suppressing the side effects.

For example, a combined use of compound (VII) with an antibacterial agent is preferable for eradicating *H. pylori.* More specifically, a combined use of the compound of the present invention with clarithromycin and/or metronidazole is preferable.

Such "other active ingredients" and a prodrug such as the compound (I) and the like of the present invention may be mixed, prepared as a single pharmaceutical composition [e.g., tablets, powders, granules, capsules (including soft capsules), liquids, injectable preparations, suppositories, sustained-release preparations, etc.], in accordance with a method known per se, and used in combination, and may also be prepared as separate preparations and administered to the same subject simultaneously or at a time interval.

### Examples

The present invention is explained in detail in the following by referring to Reference Examples and Examples. The present invention is not limited by the Examples.

In the following Reference Examples and Examples, room temperature means about 15-30°C.

¹H-NMR spectra were determined with CDCl₃, DMSO-d₆ and CD₃OD as the solvent using Varian Gemini-200 and Mercury-300; data are shown in chemical shift δ (ppm) from the internal standard tetramethylsilane.

Other symbols in the present specification mean the following.
- s:: singlet
- d:: doublet
- t:: triplet
- q:: quartet
- m:: multiplet
- br:: broad
- bs:: broad singlet
- bm:: broad multiplet
- J:: coupling constant

### Reference Example 1

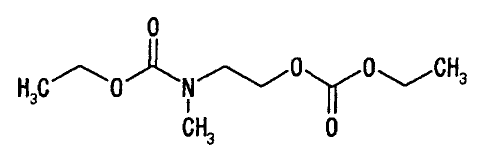

### 2-[(Ethoxycarbonyl)(methyl)amino]ethyl ethyl carbonate

To a solution (1000 mL) of 2-(methylamino)ethanol (100 g) in ethyl acetate was added pyridine (222 mL), ethyl chlorocarbonate (240 mL) was dropwise added over 2 hrs. under ice-cooling. After the completion of the dropwise addition, the reaction mixture was stirred at room temperature for 18 hrs. Water (300 mL) was added, and the ethyl acetate layer was separated and washed with 1N hydrochloric acid (200 mL) and saturated brine (200 mL). After drying over anhydrous sodium sulfate, the mixture was concentrated under reduced pressure, and the residue was distilled under reduced pressure to give the title compound (180 g) as a colorless fraction having a boiling point of 95-100°C (pressure: 0.1-0.2 mmHg).
¹H-NMR (CDCl₃) : 1.20-1.40(6H,m), 2.97(3H,s), 3.50-3.60(2H,m), 4.05-4.35(6H,m).

### Reference Example 2

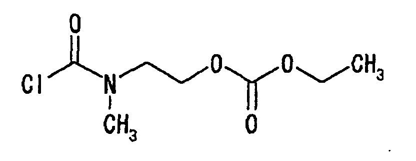

### 2-[(Chlorocarbonyl)(methyl)amino]ethyl ethyl carbonate

To a solution (1500 mL) of 2-[(ethoxycarbonyl)(methyl)amino]ethyl ethyl carbonate (150 g) obtained in Reference Example 1 in acetonitrile was added phosphorous oxychloride (200 mL) , and the mixture was refluxed for 4 days. The reaction mixture was concentrated under reduced pressure and the residue was added to a mixture of water (500 mL) - ice (700 g) - ethyl acetate (300 mL) by portions with stirring. After stirring for 1 min., saturated brine (500 mL) was added, and the mixture was extracted with ethyl acetate (500 mL). The ethyl acetate layer was washed successively with saturated brine (300 mL), a saturated aqueous sodium hydrogen carbonate solution (300 mL) and saturated brine (300 mL), dried over anhydrous sodium sulfate and concentrated under reduced pressure. The residue was distilled under reduced pressure to give the title compound (77 g) as a colorless fraction having a boiling point of 100-105°C (pressure: 0.1-0.2 mmHg).
¹H-NMR (CDCl₃) : 1.33(3H,t,J=7.2Hz), 3.12(3H×0.4,s), 3.22(3H×0.6,s), 3.68(2H×0.6,t,J=4.8Hz), 3.78(2H×0.4,t,J=4.8Hz), 4.23(2H,q,J=7.2Hz), 4.30-4.40(2H,m).

### Reference Example 3

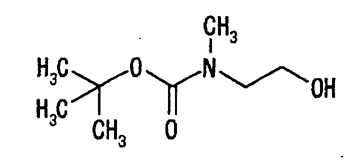

### tert-Butyl 2-hydroxyethyl(methyl)carbamate

To a mixture of 2-(methylamino)ethanol (30.04 g) and ethyl acetate (90 mL) was dropwise added a mixture of di-tert-butyl dicarbonate (87.30 g) and ethyl acetate (10 mL) under ice-cooling. After stirring at room temperature for 2 hrs., the mixture was concentrated under reduced pressure. The residue was dissolved in ethyl acetate (150 mL), washed with water (100 mL) and dried over anhydrous magnesium sulfate. The residue was concentrated under reduced pressure to give the title compound (66.19 g) as a colorless oil.
¹H-NMR(CDCl₃) : 1.47(9H,s) ,2.92(3H,s) ,3.40(2H,t,J=5.1Hz) ,3.72-3.80(2H,m).

### Reference Example 4

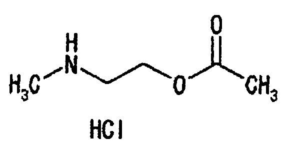

### 2-(Methylamino)ethyl acetate hydrochloride

To a mixture of 2-(methylamino)ethanol (1.50 g) and ethyl acetate (20 mL) was added di-tert-butyl dicarbonate (4.37 g) under ice-cooling, and after stirring under ice-cooling for 1.5 hrs., acetic anhydride (2.08 mL) , pyridine (1.78 mL) and 4-dimethylaminopyridine (0.12 g) were added. After stirring at room temperature for 2 hrs, the reaction mixture was washed with water (50 mL), 5% aqueous citric acid solution (50 mL) and saturated brine (50 mL). After drying over anhydrous magnesium sulfate, the residue was concentrated under reduced pressure. A 4N hydrogen chloride-ethyl acetate solution (20 mL) was added to the residue and the residue was stirred at room temperature for 2 hrs. Diethyl ether (10 mL) was added and the precipitated solid was collected by filtration and dried under reduced pressure to give the title compound (2.93 g) as a white solid.
¹H-NMR (DMSO-d₆) : 2.07(3H,s), 2.53(3H,s), 3.12-3.17(2H,m), 4.24-4.30(2H,m), 9.29(2H,br).

### Reference Example 5

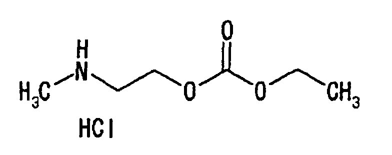

### Ethyl 2-(methylamino)ethyl carbonate hydrochloride

To a mixture of tert-butyl 2-hydroxyethyl(methyl)carbamate (1.75 g) obtained in Reference Example 3 and ethyl acetate (20 mL) were added pyridine (0.97 mL) and 4-dimethylaminopyridine (catalytic amount), and then ethyl chlorocarbonate (1.25 mL) was added dropwise. The mixture was stirred overnight at room temperature and ethyl acetate (50 mL) was added. The mixture was washed with water (50 mL), 5% aqueous citric acid solution (50 mL) and saturated brine (50 mL) and dried over anhydrous magnesium sulfate. After concentration under reduced pressure, 4N hydrogen chloride-ethyl acetate solution (10 mL) was added to the residue. After stirring at room temperature for 2 hrs., diethyl ether (10 mL) was added and the precipitated solid was collected by filtration and dried under reduced pressure to give the title compound (1.66 g) as a white solid.
¹H-NMR(DMSO-d₆) : 1.23(3H,t,J=7.1Hz), 2.54(3H,s), 3.16-3.22(2H,m), 4.15(2H,q,J=7.1Hz), 4.32-4.37(2H,m), 9.25(2H,br).

### Reference Example 6

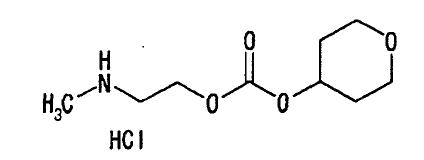

### 2-(Methylamino)ethyl tetrahydropyran-4-yl carbonate hydrochloride

To a solution (40 mL) of bis (trichloromethyl) carbonate (2.97 g) in tetrahydrofuran was added dropwise a solution (10 mL) of pyridine (2.43 mL) in tetrahydrofuran under ice-cooling. After stirring under ice-cooling for 10 min., a solution (20 mL) of tetrahydropyran-4-ol (1.91 g) in tetrahydrofuran was slowly added dropwise. The mixture was stirred at room temperature for 2 hrs. and concentrated under reduced pressure. To the residue were added ethyl acetate (50 mL) and water (50 mL). The ethyl acetate layer was separated and washed with 0.2N hydrochloric acid (20 mL) and saturated brine (50 mL) and dried over anhydrous magnesium sulfate. The residue was concentrated under reduced pressure to give chlorocarbonyl tetrahydropyran-4-yl (1.53 g). To a mixture of tert-butyl 2-hydroxyethyl(methyl)carbamate (1.40 g) obtained in Reference Example 3 and tetrahydrofuran (20 mL) was added pyridine (0.78 mL) and a solution (10 mL) chlorocarbonyl tetrahydropyran-4-yl (1.53 g) obtained above in tetrahydrofuran was added dropwise and the mixture was stirred overnight at room temperature. The reaction mixture was concentrated under reduced pressure and water (50 mL) was added. The mixture was extracted with ethyl acetate (50 mL). The ethyl acetate layer was washed with 5% aqueous citric acid solution (50 mL) and saturated brine (50 mL) and dried over anhydrous magnesium sulfate. After concentration under reduced pressure, the residue was purified by silica gel column chromatography (eluted with ethyl acetate:hexane=4:1, then 3:2). The obtained colorless oil (2.03 g) was dissolved in diethyl ether (2 mL), and 4N hydrogen chloride-ethyl acetate solution (5 mL) was added. The mixture was stirred at room temperature for 30 min. and diethyl ether (10 mL) was added. The mixture was stirred overnight. The precipitated solid was collected by filtration and dried under reduced pressure to give the title compound (1.20 g) as a white solid.
¹H-NMR (DMSO-d₆) : 1.50-1.65(2H,m), 1.87-1.98(2H,m), 2.54(3H,s), 3.20(2H,m), 3.40-3.50(2H,m), 3.74-3.83(2H,m), 4.36(2H,t,J=5.1Hz), 4,72-4.83(1H,m), 9.32(2H,br).

### Example 1

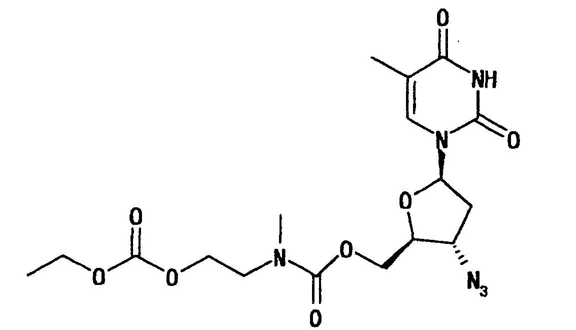

### Ethyl 2-(((((2S,3S,5R)-3-azide-5-(5-methyl-2,4-dioxo-3,4-dihydro-1(2H)-pyrimidinyl)tetrahydro-2-furanyl)methoxy)carbonyl)(methyl)amino)ethyl carbonate

To a solution of (2S,3S,5R)-3-azide-2-hydroxymethyl-5-(5-methyl-2,4-dioxo-3,4-dihydro-1(2H)-pyrimidinyl)tetrahydrofuran (0.5 g) in tetrahydrofuran (5.0 mL) were added 2-[(chlorocarbonyl)(methyl)amino]ethyl ethyl carbonate (0.436 g) obtained in Reference Example 2, triethylamine (0.391 mL) and 4-dimethylaminopyridine (0.024 g) and the mixture was stirred at 50°C for 2 hrs. The reaction mixture was extracted with ethyl acetate-water, and the organic layer was washed with saturated brine, dried over anhydrous sodium sulfate and concentrated under reduced pressure to give the title compound (0.45 g) as a colorless oil.
¹H-NMR(CDCl₃) : 1.31(3H,t,J=6.9Hz), 1.92(3H,s), 2.20-2.55(2H,m) 3.01(3H,s), 3.40-3.65(2H,m), 4.00-4-.50(8H,m), 6.11(1H,m), 7.22(1H,s), 8.70(1H,br).

### Example 2

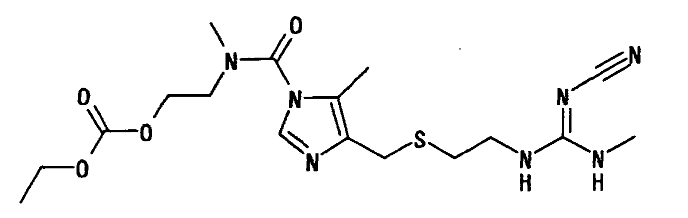

### Ethyl 2-(((4-(((2-(((cyanoamino)(methylamino)methylene)amino)ethyl)thio)methyl)-5-methyl-1H-imidazol-1-yl)carbonyl)(methyl)amino)ethyl carbonate

To a solution of 4-(((2-(((cyanoamino)(methylamino)methylene)amino)ethyl)thio)methyl)-5-methyl-1H-imidazole (0.5 g) in tetrahydrofuran (10 ml) were added 2-[(chlorocarbonyl)(methyl)amino]ethyl ethyl carbonate (0.554 g) obtained in Reference Example 2, triethylamine (0.414 mL) and 4-dimethylaminopyridine (0.024 g) and the mixture was stirred at 50°C for 18 hrs. The reaction mixture was extracted with ethyl acetate-water, and the organic layer was washed with saturated brine, dried over anhydrous sodium sulfate and concentrated under reduced pressure. The residue was purified by basic silica gel column chromatography (eluted with ethyl acetate, then ethyl acetate:methanol=10:1) to give the title compound (0.414 g) as a colorless solid.
¹H-NMR(DMSO-d₆) : 1.21(3H,t,J=7.3Hz), 2.16(3H,s), 2.61(2H,t,J=6.9Hz), 2.69(3H,d,J=4.6Hz), 2.95(3H,s), 3.33(2H,t,J=6.9Hz), 3.63(2H,t,J=5.1Hz), 3.65(2H,s), 4.11(2H,q,J=7.3Hz), 4.73(2H,t,J=5.1Hz), 7.12-7.03(2H,br), 7.78(1H,s).

### Example 3

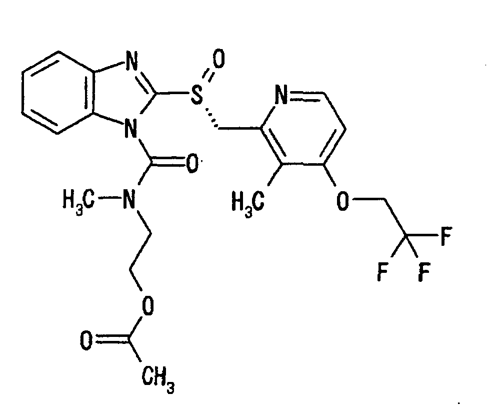

### 2-[Methyl[[(R)-2-[[[3-methyl-4-(2,2,2-trifluoroethoxy)-2-pyridyl]methyl]sulfinyl]-1H-benzimidazol-1-yl]carbonyl]amino]ethyl acetate

To a solution (30 mL) of bis(trichloromethyl)carbonate (0.50 g) in tetrahydrofuran was dropwise added a solution (1 mL) of pyridine (0.40 mL) in tetrahydrofuran under ice-cooling. After stirring under ice-cooling for 30 min., 2-(methylamino)ethyl acetate hydrochloride (0.77 g) obtained in Reference Example 4 was added. A solution (1 mL) of triethylamine (0.70 mL) in tetrahydrofuran was dropwise added, and the mixture was stirred at room temperature for 1 hr. After concentration under reduced pressure, water (50 mL) was added to the residue. The mixture was extracted with ethyl acetate (50 mL). The ethyl acetate layer was washed with saturated brine (50 mL) and dried over anhydrous magnesium sulfate. The layer was concentrated under reduced pressure, and the residue was dissolved in tetrahydrofuran (20 mL). (R)-2-[[[3-Methyl-4-(2,2,2-trifluoroethoxy)-2-pyridyl]methyl]sulfinyl]-1H-benzimidazole (1.11 g), triethylamine (0.84 mL) and 4-dimethylaminopyridine (catalytic amount) were added, and the mixture was stirred overnight at 60°C. After concentration under reduced pressure, water (50 mL) was added to the residue. The mixture was extracted with ethyl acetate (50 mL). The ethyl acetate layer was washed with saturated brine (50 mL) and dried over anhydrous magnesium sulfate. After concentration under reduced pressure, the residue was purified by basic silica gel column chromatography (eluted with ethyl acetate:hexane=1:1, then with ethyl acetate), further purified by silica gel column chromatography (eluted with ethyl acetate:hexane=2:1, then with ethyl acetate, then with acetone:ethyl acetate=1:4, then 1:1) to give the title compound (1.13 g) as a yellow amorphous solid.
¹H-NMR (CDCl₃) : 2.10(3H,s), 2.24(3H,s), 3.09(3H,bs), 3.60-4.00(2H,br), 4.25-4.50(4H,m), 4.89(1H,d,J=13.3Hz), 5.05(1H,d,J=13.3Hz), 6.65(1H,d,J=5.5Hz), 7.35-7.51(3H,m), 7.80-7.90(1H,m), 8.35(1H,d,J=5.5Hz).

### Example 4

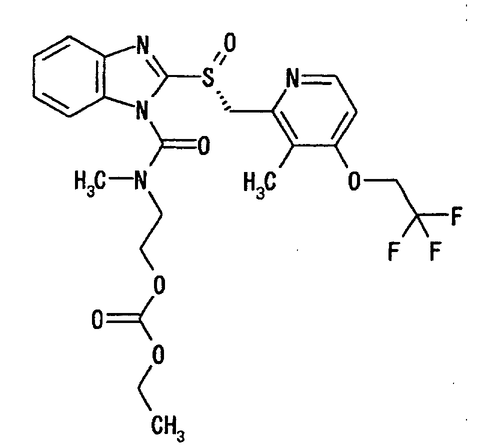

### Ethyl 2-[methyl[[(R)-2-[[[3-methyl-4-(2,2,2-trifluoroethoxy)-2-pyridyl]methyl]sulfinyl]-1H-benzimidazol-1-yl]carbonyl]amino]ethyl carbonate

To a solution (40 mL) of bis(trichloromethyl)carbonate (1.31 g) in tetrahydrofuran was dropwise added a solution (2 mL) of pyridine (1.07 mL) in tetrahydrofuran under ice-cooling. After stirring under ice-cooling for 10 min., ethyl 2-(methylamino)ethyl carbonate hydrochloride (2.02 g) obtained in Reference Example 5 was added. A solution (2 mL) of triethylamine (1.84 mL) in tetrahydrofuran was dropwise added and the mixture was stirred at room temperature for 1 hr. After concentration under reduced pressure, water (100 mL) was added to the residue, and the mixture was extracted with ethyl acetate (100 mL). The ethyl acetate layer was washed with 0.2N hydrochloric acid (50 mL) and saturated brine (100 mL) and dried over anhydrous magnesium sulfate. After concentration under reduced pressure, the residue was dissolved in tetrahydrofuran (50 mL). (R)-2-[[[3-Methyl-4-(2,2,2-trifluoroethoxy)-2-pyridyl]methyl]sulfinyl]-1H-benzimidazole (3.69 g), triethylamine (2.09 mL) and 4-dimethylaminopyridine (0.12 g) were added, and the mixture was stirred at 60°C for 6 hrs. and at room temperature for 8 hrs. After concentration under reduced pressure, water (100 mL) was added to the residue, and the mixture was extracted with ethyl acetate (100 mL). The ethyl acetate layer was washed with saturated brine (100 mL) and dried over anhydrous magnesium sulfate. After concentration under reduced pressure, the residue was purified by basic silica gel column chromatography (eluted with ethyl acetate:hexane=3:7, then ethyl acetate). Crystallization from diethyl ether and recrystallization from diethyl ether gave the title compound (3.84 g) as a colorless solid.
¹H-NMR(CDCl₃) : 1.32(3H,t,J=7.2Hz), 2.23(3H,s), 3.10(3H,bs), 3.50-4.20(2H,br), 4.22(2H,q,J=7.2Hz), 4.39(2H,q,J=7.9Hz), 4.45(2H,m), 4.80-5.15(2H,br), 6.65(1H,d,J=5.6Hz), 7.36-7.50(3H,m), 7.84(1H,d,J=7.8Hz), 8.35(1H,d,J=5.6Hz).

### Example 5

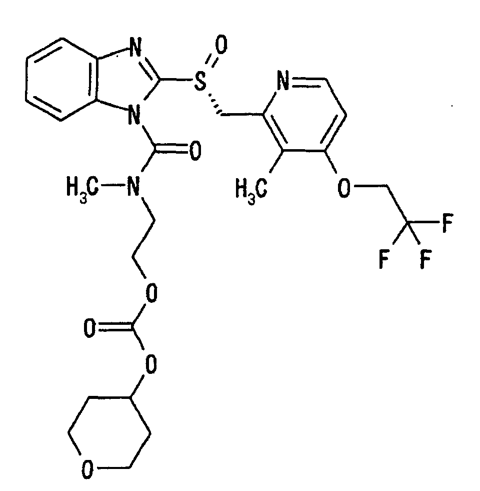

### 2-[Methyl[[(R)-2-[[[3-methyl-4-(2,2,2-trifluoroethoxy)-2-pyridyl]methyl]sulfinyl]-1H-benzimidazol-1-yl]carbonyl]amino]ethyl tetrahydropyran-4-yl carbonate

To a solution (20 mL) of bis(trichloromethyl)carbonate (0.48 g) in tetrahydrofuran was dropwise added a solution (1 mL) of pyridine (0.39 mL) in tetrahydrofuran under ice-cooling. After stirring under ice-cooling for 20 min., 2-(methylamino)ethyl tetrahydropyran-4-yl carbonate hydrochloride (0.96 g) obtained in Reference Example 6 was added. A solution (1 mL) of triethylamine (0.67 mL) in tetrahydrofuran was dropwise added, and the mixture was stirred at room temperature for 2 hrs. After concentration under reduced pressure, water (50 mL) was added to the residue. The mixture was extracted with ethyl acetate (50 mL). The ethyl acetate layer was washed with 0.2N hydrochloric acid (20 mL) and saturated brine (50 mL) and dried over anhydrous magnesium sulfate. The layer was concentrated under reduced pressure, and the residue was dissolved in tetrahydrofuran (20 mL). (R)-2-[[[3-Methyl-4-(2,2,2-trifluoroethoxy)-2-pyridyl]methyl]sulfinyl]-1H-benzimidazole (1.26 g), triethylamine (0.71 mL) and 4-dimethylaminopyridine (0.042 g) were added, and the mixture was stirred at 60°C for 6 hrs. and at room temperature for 8 hrs. After concentration under reduced pressure, water (50 mL) was added to the residue. The mixture was extracted with ethyl acetate (50 mL). The ethyl acetate layer was washed with saturated brine (50 mL) and dried over anhydrous magnesium sulfate. After concentration under reduced pressure, the residue was purified by basic silica gel column chromatography (eluted with ethyl acetate:hexane=3:7, then ethyl acetate). Crystallization from diethyl ether and recrystallization from acetone-diisopropyl ether gave the title compound (1.45 g) as a colorless solid.
¹H-NMR(CDCl₃) : 1.64-1.81(2H,m), 1.92-2.03(2H,m), 2.23(3H,s), 3.09(3H,bs), 3.40-4.30(2H,br), 3.45-3.57(2H,m), 3.87-3.97(2H,m), 4.38(2H,q,J=7.8Hz), 4.45(2H,m), 4.77-5.15(3H,m), 6.64(1H,d,J=5.7Hz), 7.35-7.50(3H,m), 7.83(1H,d,J=6.9Hz), 8.35(1H,d,J=5.7Hz).

### Example 6

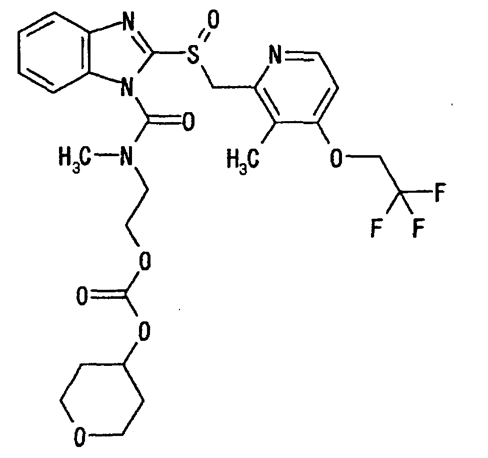

### 2-[Methyl[[2-[[[3-methyl-4-(2,2,2-trifluoroethoxy)-2-pyridyl]methyl]sulfinyl]-1H-benzimidazol-1-yl]carbonyl]amino]ethyl tetrahydropyran-4-yl carbonate

To a solution (20 mL) of bis(trichloromethyl)carbonate (0.582 g) in tetrahydrofuran was dropwise added a solution (1 mL) of pyridine (0.485 mL) in tetrahydrofuran under ice-cooling. After stirring under ice-cooling for 20 min., 2-(methylamino)ethyl tetrahydropyran-4-yl carbonate hydrochloride (1.43 g) obtained in Reference Example 6 was added. A solution (1 mL) of triethylamine (0.84 mL) in tetrahydrofuran was dropwise added, and the mixture was stirred at room temperature for 3 hrs. After concentration under reduced pressure, water (30 mL) was added to the residue, and the mixture was extracted with ethyl acetate (80 mL). The ethyl acetate layer was washed with saturated brine (20 mL), dried over anhydrous magnesium sulfate and concentrated under reduced pressure. The residue was dissolved in tetrahydrofuran (20 mL). 2-[[[3-Methyl-4-(2,2,2-trifluoroethoxy)-2-pyridyl]methyl]sulfinyl]-1H-benzimidazole (1.63 g), triethylamine (1.23 mL) and 4-dimethylaminopyridine (0.027 g) were added, and the mixture was stirred at 60°C for 17.5 hrs. After concentration under reduced pressure, water (50 mL) was added to the residue, and the mixture was extracted with ethyl acetate (120 mL). The ethyl acetate layer was washed with saturated brine (30 mL) and dried over anhydrous magnesium sulfate. After concentration under reduced pressure, the residue was purified by basic silica gel column chromatography (eluted with ethyl acetate:hexane=1:2, then 1:1), then by silica gel column chromatography (eluted with ethyl acetate:hexane=1:1, then 2:1). Crystallization from diethyl ether gave the title compound (1.23 g) as a colorless solid.
¹H-NMR(CDCl₃) : 1.64-1.81(2H,m), 1.92-2.03(2H,m), 2.23(3H,s), 3.10(3H,bs), 3.40-4.30(2H,br), 3.46-3.59(2H,m), 3.87-3.99(2H,m), 4.39(2H,q,J=7.9Hz), 4.45(2H,m), 4.77-5.15(3H,m), 6.65(1H,d,J=5.4Hz), 7.35-7.50(3H,m), 7.85(1H,m), 8.36(1H,d,J=5.4Hz).

### Example 7

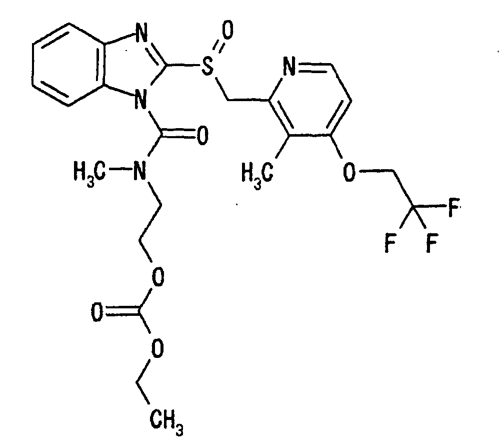

### Ethyl 2-[methyl[[2-[[[3-methyl-4-(2,2,2-trifluoroethoxy)-2-pyridyl]methyl]sulfinyl]-1H-benzimidazol-1-yl]carbonyl]amino]ethyl carbonate

To a solution (20 mL) of bis(trichloromethyl)carbonate (0.582 g) in tetrahydrofuran was dropwise added a solution (1 mL) of pyridine (0.485 mL) in tetrahydrofuran under ice-cooling. After stirring under ice-cooling for 30 min., ethyl 2-(methylamino)ethyl carbonate hydrochloride (1.10 g) obtained in Reference Example 5 was added. A solution (1 mL) of triethylamine (0.84 mL) in tetrahydrofuran was dropwise added, and the mixture was stirred at room temperature for 3 hrs. After concentration under reduced pressure, water (30 mL) was added to the residue, and the mixture was extracted with ethyl acetate (80 mL). The ethyl acetate layer was washed with saturated brine (30 mL) and dried over anhydrous magnesium sulfate. The layer was concentrated under reduced pressure, and the residue was dissolved in tetrahydrofuran (20 mL). 2-[[[3-Methyl-4-(2,2,2-trifluoroethoxy)-2-pyridyl]methyl]sulfinyl]-1H-benzimidazole (1.63 g), triethylamine (1.23 mL), 4-dimethylaminopyridine (0.054 g) were added, and the mixture was stirred at 60°C for 14 hrs. After concentration under reduced pressure, water (40 mL) was added to the residue, and the mixture was extracted with ethyl acetate (100 mL). The ethyl acetate layer was washed with saturated brine (30 mL), and dried over anhydrous magnesium sulfate. After concentration under reduced pressure, the residue was purified by basic silica gel column chromatography (eluted with ethyl acetate:hexane=1:2, then 1:1), and then by silica gel column chromatography (eluted with ethyl acetate:hexane=1:1, then 2:1) to give the title compound (1.27 g) as a yellow amorphous solid.
¹H-NMR(CDCl₃) : 1.32(3H,t,J=7.1Hz), 2.23(3H,s), 3.09(3H,bs), 3.50-4.76(4H,br), 4.21(2H,q,J=7.1Hz), 4.38(2H,q,J=7.9Hz), 4.84-5.14(2H,m), 6.64(1H,d,J=5.6Hz), 7.36-7.46(3H,m), 7.83(1H,d,J=7.2Hz), 8.34(1H,d,J=5.6Hz)

### Example 8

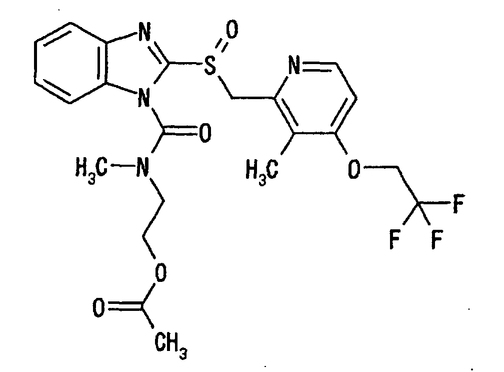

### 2-[Methyl[[2-[[[3-methyl-4-(2,2,2-trifluoroethoxy)-2-pyridyl]methyl]sulfinyl]-1H-benzimidazol-1-yl]carbonyl]amino]ethyl acetate

To a solution (20 mL) of bis(trichloromethyl)carbonate (0.582 g) in tetrahydrofuran was dropwise added a solution (1 mL) of pyridine (0.485 mL) in tetrahydrofuran under ice-cooling. After stirring under ice-cooling for 1 hr., 2-(methylamino)ethyl acetate hydrochloride (0.922 g) obtained in Reference Example 4 was added. A solution (1 mL) of triethylamine (0.84 mL) in tetrahydrofuran was dropwise added, and the mixture was stirred at room temperature for 2.5 hrs. After concentration under reduced pressure, water (40 mL) was added to the residue, and the mixture was extracted with ethyl acetate (80 mL). The ethyl acetate layer was washed with saturated brine (25 mL) and dried over anhydrous magnesium sulfate. After concentration under reduced pressure, the residue was dissolved in tetrahydrofuran (15 mL). 2-[[[3-Methyl-4-(2,2,2-trifluoroethoxy)-2-pyridyl]methyl]sulfinyl]-1H-benzimidazole (1.10 g), triethylamine (0.84 mL) and 4-dimethylaminopyridine (0.036 g) were added, and the mixture was stirred at 60°C for 4.5 hrs. After concentration under reduced pressure, water (40 mL) was added to the residue, and the mixture was extracted with ethyl acetate (80 mL). The ethyl acetate layer was washed with saturated brine (30 mL) and dried over anhydrous magnesium sulfate. After concentration under reduced pressure, the residue was purified by silica gel column chromatography (eluted with ethyl acetate:hexane=1:1, then 2:1) to give the title compound (1.18 g) as a colorless solid.
¹H-NMR (CDCl₃) : 2.10(3H,s), 2.24,(3H,s), 3.09(3H,bs), 3.60-4.00(2H,br), 4.25-4.50(2H,m), 4.38(2H, q,J=7.8Hz), 4.84-5.18(2H,m), 6.64(1H,d,J=5.6Hz), 7.36-7.48(3H,m), 7.85(1H,d,J=7.8Hz) 8.35(1H,d,J=5.6Hz).

### Example 9

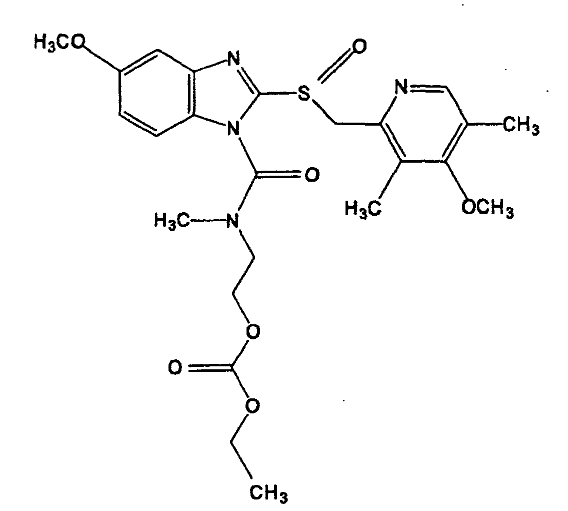

### Ethyl 2-[[[5-methoxy-2-[[(4-methoxy-3,5-dimethyl-2-pyridyl)methyl]sulfinyl]-1H-benzimidazol-1-yl]carbonyl](methyl)amino]ethyl carbonate

To a solution (10 mL) of bis(trichloromethyl)carbonate (0.291 g) in tetrahydrofuran was dropwise added a solution (1 mL) of pyridine (0.243 mL) in tetrahydrofuran under ice-cooling. After stirring under ice-cooling for 1 hr., ethyl 2-(methylamino)ethyl carbonate hydrochloride (0.551 g) obtained in Reference Example 5 was added. A solution (1 mL) of triethylamine (0.418 mL) in tetrahydrofuran was dropwise added, and the mixture was stirred at room temperature for 2 hrs. After concentration under reduced pressure, water (15 mL) was added to the residue, and the mixture was extracted with ethyl acetate (50 mL). The ethyl acetate layer was washed with saturated brine (15 mL) and dried over anhydrous magnesium sulfate. After concentration under reduced pressure, the residue was dissolved in tetrahydrofuran (10 mL). 5-Methoxy-2-[[(4-methoxy-3,5-dimethyl-2-pyridyl)methyl]sulfinyl]-1H-benzimidazole (0.817 g), triethylamine (0.661 mL) and 4-dimethylaminopyridine (0.012 g) were added, and the mixture was stirred at 60°C for 12 hrs. After concentration under reduced pressure, water (20 mL) was added to the residue, and the mixture was extracted with ethyl acetate (50 mL). The ethyl acetate layer was washed with saturated brine (15 mL) and dried over anhydrous magnesium sulfate. After concentration under reduced pressure, the residue was purified by basic silica gel column chromatography (eluted with ethyl acetate:hexane=1:2, then 1:1) to give a 3:2 mixture (0.92 g) of the title compound and ethyl 2-[[[6-methoxy-2-[[(4-methoxy-3,5-dimethyl-2-pyridyl)methyl]sulfinyl]-1H-benzimidazol-1-yl]carbonyl](methyl)amino]ethyl carbonate as a pale-yellow amorphous solid.
¹H-NMR(CDCl₃) : 1.27-1.34(3H,m), 2.10-2.30(3H,m), 2.23(3H,s), 2.99-3.23(3H,m), 3.40-3.85(2H,m), 3.69(6/5H,s), 3.71(9/5H,s), 3.86(6/5H,s), 3.88(9/5H,s), 4.14-4.25(2H,m), 4.38-4.60(2H,m), 4.82-5.06(2H,m), 6.92-7.08(7/5H,m), 7.33(3/5H,d,J=9.0Hz), 7.66(1H,m), 8.21(1H,s).

### Example 10

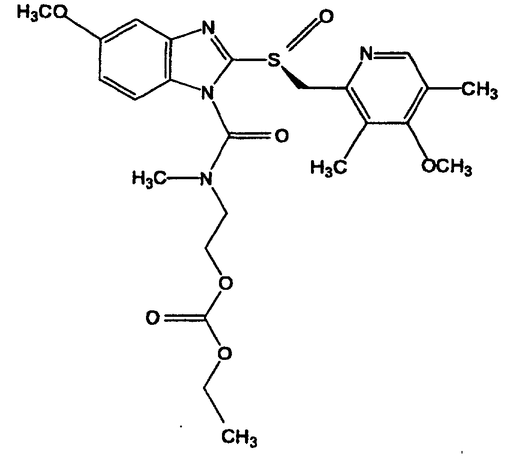

### Ethyl 2-[[[(S)-5-methoxy-2-[[(4-methoxy-3,5-dimethyl-2-pyridyl)methyl]sulfinyl]-1H-benzimidazol-1-yl]carbonyl](methyl)amino]ethyl carbonate

To a solution (10 mL) of (S)-5-methoxy-2-[[(4-methoxy-3,5-dimethyl-2-pyridyl)methyl]sulfinyl]-1H-benzimidazole (1.34 g) synthesized by the method described in Example 1 of Japanese Patent Application under PCT laid-open under kohyo No. 10-504290 in tetrahydrofuran were added 2-[(chlorocarbonyl)(methyl)amino]ethyl ethyl carbonate (0.9 mL) obtained in Reference Example 2, triethylamine (1.08 mL) and 4-dimethylaminopyridine (0.010 g), and the mixture was stirred at 60°C for 6 hrs. After concentration under reduced pressure water (30 mL) was added to the residue and the mixture was extracted with ethyl acetate (50 mL). The ethyl acetate layer was washed with saturated brine (15 mL) and dried over anhydrous magnesium sulfate. After concentration under reduces pressure, the residue was purified by basic silica gel column chromatography (eluted with ethyl acetate:hexane=1:2, then 1:1) to give a 3:2 mixture (0.92 g) of the title compound and ethyl 2-[[[(S)-6-methoxy-2-[[(4-methoxy-3,5-dimethyl-2-pyridyl)methyl]sulfinyl]-1H-benzimidazol-1-yl]carbonyl](methyl)amino]ethyl carbonate as a pale-yellow amorphous solid.
¹H-NMR(CDCl₃) : 1.25-1.34(3H,m), 2.10-2.30(3H,m), 2.23(3H,s), 2.99-3.23(3H,m), 3.40-3.85(2H,m), 3.69(6/5H,s), 3.71(9/5H,s), 3.86(6/5H,s), 3.88(9/5H,s), 4.14-4.25(2H,m), 4.38-4.60(2H,m), 4.79-5.05(2H,m), 6.92-7.08(7/5H,m), 7.33(3/5H,d,J=9.3Hz), 7.65(1H,m), 8.21(1H,s).

### Example 11

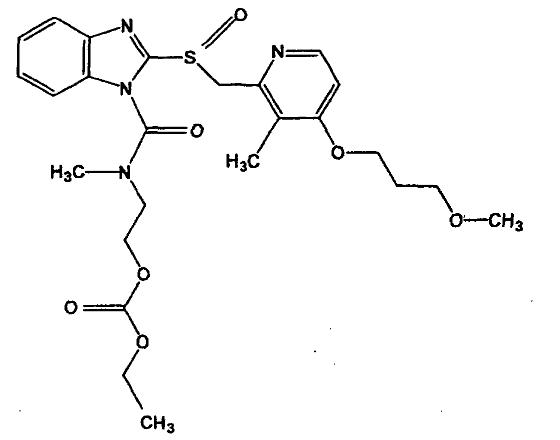

### Ethyl 2-[[[2-[[[4-(3-methoxypropoxy)-3-methyl-2-pyridyl]methyl]sulfinyl]-1H-benzimidazol-1-yl]carbonyl](methyl)amino]ethyl carbonate

To a solution (10 mL) of bis(trichloromethyl)carbonate (0.291 g) in tetrahydrofuran was dropwise added a solution (1 mL) of pyridine (0.243 mL) in tetrahydrofuran under ice-cooling. After stirring under ice-cooling for 30 min., ethyl 2-(methylamino)ethyl carbonate hydrochloride (0.551 g) obtained in Reference Example 5 was added. A solution (1 mL) of triethylamine (0.418 mL) in tetrahydrofuran was dropwise added, and the mixture was stirred at room temperature for 2.5 hrs. After concentration under reduced pressure, water (15 mL) was added to the residue, and the mixture was extracted with ethyl acetate (50 mL). The ethyl acetate layer was washed with saturated brine (15 mL) and dried over anhydrous magnesium sulfate. After concentration under reduced pressure, the residue was dissolved in tetrahydrofuran (10 mL). 2-[[[4-(3-Methoxypropoxy)-3-methyl-2-pyridyl]methyl]sulfinyl]-1H-benzimidazole (0.723 g), triethylamine (0.528 mL) and 4-dimethylaminopyridine (0.012 g) were added, and the mixture was stirred at 60°C for 17 hrs. After concentration under reduced pressure, water (40 mL) was added to the residue, and the mixture was extracted with ethyl acetate (80 mL). The ethyl acetate layer was washed with saturated brine (15 mL) and dried over anhydrous magnesium sulfate. After concentration under reduced pressure, the residue was purified by basic silica gel column chromatography (eluted with ethyl acetate:hexane=1:2), then by silica gel column chromatography (eluted with ethyl acetate:hexane=1:1, then ethyl acetate) to give the title compound (0.44 g) as a colorless amorphous solid.
¹H-NMR(CDCl₃) : 1.31(3H,t,J=7.1Hz), 2.05(2H,m), 2.18(3H,s), 3.08(3H,bs), 3.34(3H,s), 3.54(2H,t,J=6.1Hz), 3.61-4.01(2H,m), 4.08(2H,t,J=6.3Hz), 4.21(2H,t,J=7.1Hz), 4.38-4.54(2H,m), 4.81-5.12(2H,m), 6.68(1H,d,J=5.6Hz), 7.34-7.48(3H,m), 7.83(1H,d,J=7.8Hz), 8.27(1H,d,J=5.6Hz).

### Example 12

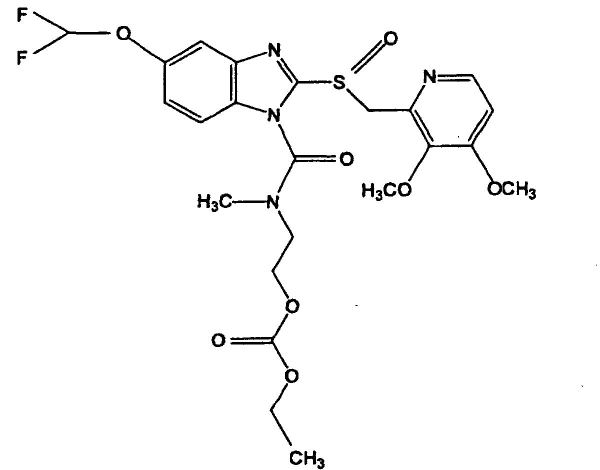

### 2-[[[5-(Difluoromethoxy)-2-[[(3,4-dimethoxy-2-pyridyl)methyl]sulfinyl]-1H-benzimidazol-1-yl]carbonyl](methyl)amino]ethyl ethyl carbonate

To a solution (8 mL) of bis(trichloromethyl)carbonate (0.174 g) in tetrahydrofuran was dropwise added a solution (1 mL) of pyridine (0.146 mL) in tetrahydrofuran under ice-cooling. After stirring under ice-cooling for 1 hr., ethyl 2-(methylamino)ethyl carbonate hydrochloride (0.330 g) obtained in Reference Example 5 was added. A solution (1 mL) of triethylamine (0.250 mL) in tetrahydrofuran was dropwise added, and the mixture was stirred at room temperature for 3 hrs. After concentration under reduced pressure, water (10 mL) was added to the residue, and the mixture was extracted with ethyl acetate (30 mL). The ethyl acetate layer was washed with saturated brine (10 mL) and dried over anhydrous magnesium sulfate. After concentration under reduced pressure, the residue was dissolved in tetrahydrofuran (8 mL). 5-(Difluoromethoxy)-2-[[(3,4-dimethoxy-2-pyridyl)methyl]sulfinyl]-1H-benzimidazole (0.432 g), triethylamine (0.279 mL) and 4-dimethylaminopyridine (0.008 g) were added, and the mixture was stirred at 60°C for 17.5 hrs. After concentration under reduced pressure, water (20 mL) was added to the residue, and the mixture was extracted with ethyl acetate (50 mL). The ethyl acetate layer was washed with saturated brine (10 mL) and dried over anhydrous magnesium sulfate. After concentration under reduced pressure, the residue was purified by basic silica gel column chromatography (eluted with ethyl acetate:hexane=1:2, then 1:1), then by silica gel column chromatography (eluted with ethyl acetate:hexane=2:1, then ethyl acetate) to give a 1:1 mixture (0.09 g) of the title compound and 2-[[[6-(difluoromethoxy)-2-[[(3,4-dimethoxy-2-pyridyl)methyl]sulfinyl]-1H-benzimidazol-1-yl]carbonyl]methylamino]ethyl ethyl carbonate as a pale-yellow amorphous solid.
¹H-NMR(CDCl₃) : 1.31(3H,t,J=7.2Hz), 3.06(3H,s), 3.42-3.98(2H,m), 3.87(3H,s), 3.90(3H,s), 4.21(2H,q,J=7.2Hz), 4.36-4.54(2H,m), 4.90(1H,d,J=13.2Hz) 4.98(1H,d,J=13.2Hz), 6.54(0.5H,t,J=73.5Hz), 6.61(0.5H,t,J=73.5Hz), 6.78(1H,d,J=5.3Hz), 7.15-7.25(1.5H,m), 7.44(0.5H,d,J=9.0Hz), 7.59(0.5H,s), 7.80(0.5H,d,J=9.0Hz), 8.17(1H,d,J=5.3Hz).

### Example 13

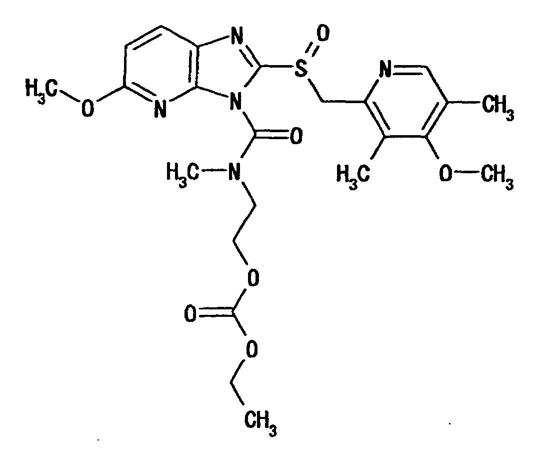

Ethyl 2-[[[5-methoxy-2-[[(4-methoxy-3,5-dimethyl-2-pyridyl)methyl]sulfinyl]-3H-imidazo[4,5-b]pyridin-3-yl]carbonyl](methyl)amino]ethyl carbonate

To a solution (20 mL) of bis (trichloromethy2) carbonate (0.582 g) in tetrahydrofuran was dropwise added a solution (1 mL) of pyridine (0.485 mL) in tetrahydrofuran under ice-cooling. After stirring under ice-cooling for 30 min., ethyl 2-(methylamino)ethyl carbonate hydrochloride (1.10 g) obtained in Reference Example 5 was added. A solution (1 mL) of triethylamine (0.84 mL) in tetrahydrofuran was added dropwise and the mixture was stirred at room temperature for 2.5 hrs. After concentration under reduced pressure, water (30 mL) was added to the residue and the mixture was extracted with ethyl acetate (80 mL). The ethyl acetate layer was washed with saturated brine (30 mL) and dried over anhydrous magnesium sulfate. The residue was concentrated under reduced pressure and dissolved in tetrahydrofuran (20 mL). 5-Methoxy-2-[[(4-methoxy-3,5-dimethyl-2-pyridyl)methyl]sulfinyl]-1H-imidazo[4,5-b]pyridine (1.44 g) synthesized according to the method described in JP-A-63-146882, trimethylamine (1.16 mL) and 4-dimethylaminopyridine (0.049 g) were added, and the mixture was stirred at 60°C for 6 hrs. After concentration under reduced pressure, water (30 mL) was added to the residue and the mixture was extracted with ethyl acetate (100 mL). The ethyl acetate layer was washed with saturated brine (30 mL) and dried over anhydrous magnesium sulfate. After concentration under reduced pressure, the residue was purified by basic silica gel column chromatography (eluted with ethyl acetate:hexane=1:2, then 1:1). Crystallization from diethyl ether gave the title compound (0.721 g) as a colorless solid.
¹H-NMR(CDCl₃) : 1.25-1.34(3H,m), 2.23(6H,s), 3.15, 3.32 (total 3H,s), 3.72(3H,s), 3.90-4.53(9H,m), 4.86(1H,d,J=13.4Hz), 4.95(1H,d,J=13.4Hz), 6.79(1H,d,J=8.7Hz), 7.95(1H,d,J=8.7Hz) 8.22(1H, s).

### Example 14

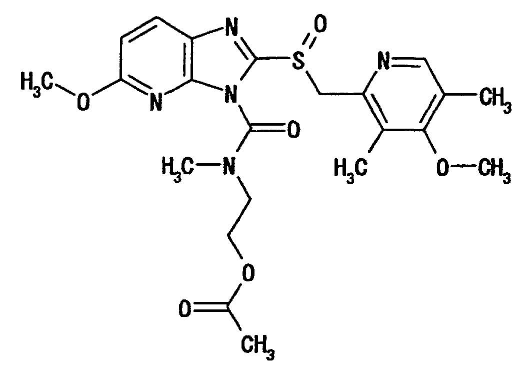

### 2-[[[5-Methoxy-2-[[(4-methoxy-3,5-dimethyl-2-pyridyl)methyl]sulfinyl]-3H-imidazo[4,5-b]pyridin-3-yl]carbonyl](methyl)amino]ethyl acetate

To a solution (20 mL) of bis(trichloromethyl)carbonate (0.582 g) in tetrahydrofuran was dropwise added a solution (1 mL) of pyridine (0.485 mL) in tetrahydrofuran under ice-cooling. After stirring under ice-cooling for 30 min., 2-(methylamino)ethyl acetate hydrochloride (0.922 g) obtained in Reference Example 4 was added. A solution (1 mL) of triethylamine (0.84 mL) in tetrahydrofuran was added dropwise and the mixture was stirred at room temperature for 2 hrs. After concentration under reduced pressure, water (30 mL) was added to the residue, and the mixture was extracted with ethyl acetate (80 mL). The ethyl acetate layer was washed with saturated brine (30 mL) and dried over anhydrous magnesium sulfate. The layer was concentrated under reduced pressure, and the residue was dissolved in tetrahydrofuran (10 mL). 5-methoxy-2-[[(4-methoxy-3,5-dimethyl-2-pyridyl)methyl]sulfinyl]-1H-imidazo[4,5-b]pyridine (0.85 g) synthesized by the method described in JP-A-63-146882, triethylamine (0.70 mL) and 4-dimethylaminopyridine (0.025 g) were added and the mixture was stirred at 60°C for 5 hrs. After concentration under reduced pressure and water (30 mL) was added to the residue and the mixture was extracted with ethyl acetate (90 mL). The ethyl acetate layer was washed with saturated brine (30 mL) and dried over anhydrous magnesium sulfate. After concentration under reduced pressure, the residue was purified by basic silica gel column chromatography (eluted with ethyl acetate:hexane=1:2, then 1:1). Crystallization from diethyl ether gave the title compound (0.173 g) as a colorless solid.
¹H-NMR(CDCl₃) : 2.04, 2.09 (total 3H,s), 2.24(6H,s), 3.13, 3.30 (total 3H,s), 3.45-3.97(2H,m), 3.72(3H,s), 3.97(3H,s), 4.15-4.50(2H,m), 4.85(1H,d,J=13.1Hz), 4.96(1H,d,J=13.1Hz), 6.80(1H,d,J=8.9Hz), 7.96(1H,d,J=8.9Hz), 8.22(1H,s).

### Preparation Example

According to the following formulation and using a centrifugal rolling granulator, a dusting powder consisting of the remaining components is coated on sucrose·starch spherical granules while spraying a hydroxypropyl cellulose solution, thereby producing spherical granules, which spherical granules are vacuum dried and passed through a round sieve to give granules.

| composition in 300 mg of granules | (mg) |
|---|---|
| sucrose·starch spherical granules | 110.0 |
| compound of Example 1 | 30.0 |
| magnesium carbonate | 22.4 |
| purified sucrose | 59.8 |
| corn starch | 36.4 |
| low substituted hydroxypropyl cellulose | 40.0 |
| hydroxypropyl cellulose | 1.4 |
| total | 300.0 |

### Industrial Applicability

According to the present invention, the development of a prodrug based on the modification of a ring-constituting nitrogen atom of a wide range of pharmaceutical compounds having a nitrogen-containing heterocycle has been made possible. Moreover, since the present invention is applicable to the development of a prodrug based on the modification of a functional group having an eliminatable proton, such as a hydroxyl group, an amino group, an amide group and the like, it provides a prodrug of a therapeutic drug or a pharmaceutical compound having a pharmacological action, which generally has such an eliminatable proton, and a means thereof.

The present invention provides a prodrug of an existing therapeutic drug or a pharmaceutical compound having a pharmacological action, and a means therefor. Forming such a prodrug affords improvement of chemical stability, improvement of absorbability, regulation of the level of pharmacological action, prolongation of pharmacological action, reduction of side effects, improvement of bad taste, reduction of irritation, expansion of selectivity of prescription of preparation, improvement of administration route, small sizing of preparation, low cost of preparation and the like.

This application is based on patent application Nos. 2002-175086 and 2003-41085 filed in Japan, the contents of which are hereby incorporated by reference.

## Claims

1. A prodrug compound having, as a modification group to be eliminated from the prodrug, a group represented by the formula: 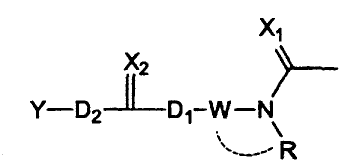 wherein
X₁ and X₂ are each an oxygen atom or a sulfur atom,
W is a chain divalent hydrocarbon group optionally having substituent(s) or a divalent group represented by the formula:
-W₁-Z-W₂-
wherein W₁ and W₂ are each a chain divalent hydrocarbon group or a bond, Z is a divalent hydrocarbon ring group optionally having substituent(s), a divalent heterocyclic group optionally having substituent(s), an oxygen atom, SOₙ wherein n is 0, 1 or 2, or >N-E wherein E is a hydrogen atom, a hydrocarbon group optionally having substituent(s), a heterocyclic group optionally having substituent(s), a lower alkanoyl group, a lower alkoxycarbonyl group, an aralkyloxycarbonyl group, a thiocarbamoyl group, a lower alkylsulfinyl group, a lower alkylsulfonyl group, a sulfamoyl group, a mono-lower alkylsulfamoyl group, a di-lower alkylsulfamoyl group, an arylsulfamoyl group, an arylsulfinyl group, an arylsulfonyl group, an arylcarbonyl group or a carbamoyl group optionally having substituent(s), and when Z is an oxygen atom, SOₙ or >N-E, W₁ and W₂ are each a chain divalent hydrocarbon group,
R is a hydrogen atom, a hydrocarbon group optionally having substituent(s) or a heterocyclic group optionally having substituent(s), and
R and W may be bonded to each other when R is not a hydrogen atom,
D₁ and D₂ are each a bond, an oxygen atom, a sulfur atom or >NR₁ wherein R₁ is a hydrogen atom or a hydrocarbon group optionally having substituent(s), except for when both D₁ and D₂ are bonds, and
Y is a hydrocarbon group optionally having substituent(s) or a heterocyclic group optionally having substituent(s).

2. The compound of claim 1, which is a compound represented by the formula (I): 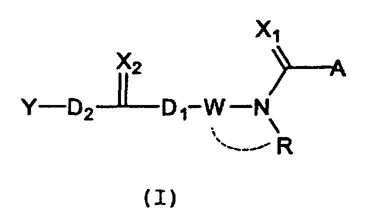 wherein A is a group remaining from elimination of hydrogen from a parent compound H-A of a prodrug having a group capable of bonding to a carbon atom of a modification group eliminatable from a prodrug, via a carbon-oxygen bond, a carbon-sulfur bond or a carbon-nitrogen bond, and other symbols are as defined in claim 1,
or a salt thereof.

3. The compound of claim 1, wherein R is a hydrocarbon group optionally having substituent(s) or a heterocyclic group optionally having substituent(s).

4. The compound of claim 1, wherein Z is a divalent hydrocarbon ring group optionally having substituent(s) or a divalent heterocyclic group optionally having substituent(s).

5. The compound of claim 1, wherein X₁ and X₂ are each an oxygen atom.

6. The compound of claim 1, wherein D₁ and D₂ are each a bond or an oxygen atom, except for when both D₁ and D₂ are bonds.

7. The compound of claim 1, wherein W is a chain divalent hydrocarbon group optionally having substituent(s).

8. The compound of claim 1, wherein W is an ethylene group.

9. The compound of claim 1, wherein R is a C₁₋₆ hydrocarbon group optionally having substituent(s).

10. The compound of claim 1, wherein Y is a C₁₋₆ hydrocarbon group optionally having substituent(s) or a saturated heterocyclic group optionally having substituent(s), which contains, as ring-constituting atom, 1 to 4 heteroatom(s) selected from oxygen atom, nitrogen atom and sulfur atom.

11. The compound of claim 1, wherein X₁ and X₂ are each an oxygen atom, D₁ and D₂ are each a bond or an oxygen atom except for when both D₁ and D₂ are bonds, W is an ethylene group, R is a C₁₋₆ alkyl group, and Y is a C₁₋₆ hydrocarbon group optionally having substituent(s) or a saturated oxygen-containing heterocyclic group optionally having substituent(s), which may further contain, as ring-constituting atom, 1 to 3 heteroatom(s) selected from oxygen atom, nitrogen atom and sulfur atom.

12. The compound of claim 1, which is a compound represented by the formula (II): 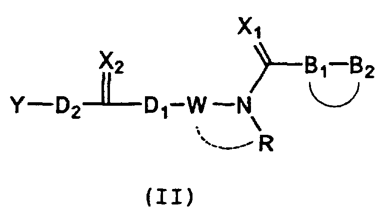 wherein -B₁-B₂ is a group remaining from elimination of hydrogen from a pharmaceutical compound H-B₁-B₂ wherein H-B₁- is a hydroxyl group, a thiol group, an amide group or an optionally fused, nitrogen-containing heterocycle optionally having substituent(s), which is capable of bonding to a carbon atom of a modification group eliminatable from a prodrug, via a carbon-oxygen bond, a carbon-sulfur bond or a carbon-nitrogen bond, and other symbols are as defined in claim 1, or a salt thereof.

13. The compound of claim 12, wherein B₁ is an optionally fused, nitrogen-containing heterocyclic group optionally having substituent(s), which is capable of bonding to a carbon atom of a modification group eliminatable from a prodrug, via a carbon-nitrogen bond.

14. The compound of claim 13, wherein the nitrogen-containing heterocyclic group represented by B₁ is a 5 or 6-membered aromatic heterocyclic group containing 1 to 4 nitrogens.

15. The compound of claim 14, wherein the aromatic heterocycle in the 5 or 6-membered aromatic heterocyclic group containing 1 to 4 nitrogens, which is represented by B₁, is imidazole, pyrrole, pyrazole, isoxazole, oxazole, thiazole or triazole.

16. (1) A production method of the compound of claim 2, which comprises reacting a pharmaceutical compound having an eliminatable proton (H) represented by the formula (III):
H-A (III)
or a salt thereof with a compound represented by the formula (IV): 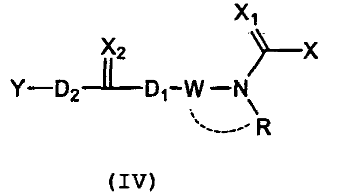 wherein X is a leaving group, and other symbols are as defined in claim 1, or a salt thereof, or a compound of the formula (V) : 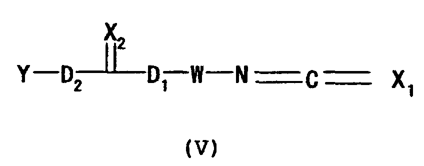 wherein each symbol is as defined in claim 1, or a salt thereof.

17. A compound represented by the formula (VI): 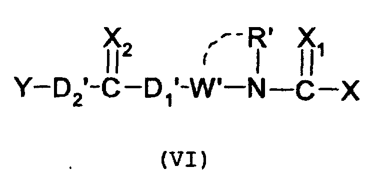 wherein X₁ and X₂ are each an oxygen atom or a sulfur atom, W' is a chain divalent hydrocarbon group having 2 or more carbon atoms and optionally having substituent(s), or a divalent group represented by the formula:
-W₁'-Z'-W₂'-
wherein W₁' and W₂' are each a chain divalent hydrocarbon group or bond, Z' is a divalent hydrocarbon ring group optionally having substituent(s) or a divalent heterocyclic group optionally having substituent(s), R' is a hydrocarbon group optionally having substituent(s) or a heterocyclic group optionally having substituent(s), R' is optionally bonded to W', D₁' is an oxygen atom or a sulfur atom and D₂' is an oxygen atom, or D₁' is a sulfur atom and D₂' is a bond, Y is a hydrocarbon group optionally having substituent(s) or a heterocyclic group optionally having substituent(s), and X is a leaving group, or a salt thereof.

18. Use of a compound represented by the formula (IV): 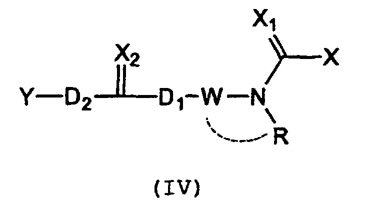 wherein X is a leaving group, and other symbols are as defined in claim 1, for the production of a prodrug compound or a salt thereof.

19. Use of a compound of the formula (V): 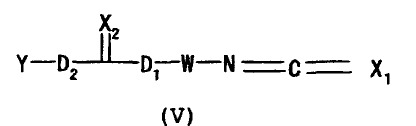 wherein each symbol is as defined in claim 1, for the production of a prodrug compound or a salt thereof.
